(19)

Europäisches Patentamt

European Patent Office

Office européen des brevets

(11) **EP 1 911 766 A1**

(12) **EUROPEAN PATENT APPLICATION**

(43) Date of publication:
**16.04.2008 Bulletin 2008/16**

(21) Application number: **06090190.7**

(22) Date of filing: **13.10.2006**

(51) Int Cl.:
*C07K 16/00* (2006.01)   *C07K 16/30* (2006.01)
*C12N 5/10* (2006.01)

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK RS**

(71) Applicant: **GLYCOTOPE GMBH
13125 Berlin (DE)**

(72) Inventor: **Goletz, Steffen, Dr.
16548 Glienicke (DE)**

(74) Representative: **Schönbohm, Carla et al
König Szynka Tilmann von Renesse
Patentanwälte Partnerschaft
Lohengrinstrasse 11
40549 Düsseldorf (DE)**

Remarks:
The biological material has been deposited with
DSMZ under number(s) DSM ACC2605, DSM
ACC2606, DSM ACC2806, DSM ACC2807

(54) **Use of human cells of myeloid leukaemia origin for expression of antibodies**

(57)   The application provides biotechnologically favourable methods for the production of antibody molecule compositions having increased activity and/or increased yield and/or improved homogeneity and fully human glycosylation. The application further provides biotechnologically favourable methods for the production of protein molecule compositions having increased activity and/or increased yield and/or improved homogeneity and fully human glycosylation. It further provides novel host cells, nucleic acids, and molecule compositions.

EP 1 911 766 A1

**Description**

SUMMARY

The invention provides biotechnologically favourable methods for the production of antibody molecule compositions having increased activity and/or increased yield and/or improved homogeneity and fully human glycosylation. The invention further provides biotechnologically favourable methods for the production of protein molecule compositions having increased activity and/or increased yield and/or improved homogeneity and fully human glycosylation. It further provides novel host cells, nucleic acids, and molecule compositions.

INTRODUCTION

Antibodies are major tools for diagnosis and research, and likely to become the largest family of therapeutics. Over ten recombinant antibody therapeutics are on the market and hundreds in clinical development. A key feature and challenge for the industry is the production of recombinant antibodies ("rMAbs") and productivity, cost, homogeneity, and antibody activity are key issues which remain to be optimised. All therapeutic rMAbs on the market have been produced in the rodent cell lines from hamster (CHO) and mice (NS0 or Sp2/0). By far most of the rMABs in development are produced in rodent cells, and others are under development, however, none has been sufficient for optimising productivity, cost, homogeneity, and antibody activity. Glycosylation is a key issue in the production of high yields of homogenous and potent rMAbs which poses a series of critical problems for the production of rMAbs. Each current production cell line offers a series of different challenges and problems which are largely due to the complexity and species, tissue and site specificity of the glycosylation [Review: Royston Jefferis, CCE IX: Glycosylation of Recombinant Antibody Therapeutics; Biotechnol.Prog. 2005, 21,11-16]. Therefore the optimisation of production systems in respect to glycosylation remains one of the key aspects for optimisation.

The present invention provides new expression systems based on human cells of myeloid leukaemia origin which surprisingly improve the production of antibodies in respect to antibody activity, yield and/or homogeneity.

BACKGROUND ART

Various classes of antibodies are present in human and most mammalian, namely IgG, IgM, IgE, IgA, IgD. While molecules of all antibody classes are used in diagnostics or as research tools, the majority of antibody based therapeutics on the market and under development are IgG and to a lesser extend IgM. The human IgG class is further classified into four subclasses, IgG1, IgG2, IgG3 and IgG4. The basic structure of an IgG molecule consists of two light chains and two heavy chains comprising two Fab regions each with one variable domain comprising the antigen-binding site and one constant domain, one Fc region comprising further constant domains and the interaction sites for ligands, and the flexible hinge region which connects Fab and Fc regions. Antibodies can exist as whole molecules or as antibody fragments such as Fab fragments or single chain Fv which comprise the two variable regions of the heavy and light chain connected via a linker. Recombinant antibodies for therapeutic use are most often chimaeric, humanized or so-called fully human protein sequences in order to reduce the immunogenicity of antibodies. However, truly fully human molecules has not be readily achieved since the posttranslational modifications such as glycosylation is not human and can differ from those modifications found on human antibodies in human sera.

The activity of an antibody can be due to and influenced by a combination of effects. On one hand side the antibody has a certain specificity which is mediated by the variable region of the antibody ("V region") located in the Fab region wherein certain sequences, the CDR regions, of the V region play a key role in determining the particular specificity and affinity of an antibody and vary from antibody to antibody. The affinity of an antibody describes the strength and kinetics of the binding of a single binding region of an antibody to its epitope. Since the various classes and subclasses of antibodies carry between two and ten identical variable regions in one antibody molecule, the strength and kinetics of the binding of an antibody is influenced by the number of binding sites available for binding to and the accessibility of epitopes on the target antigen or cell and is expressed in its avidity. Another important factor for the quality of an antibody for its use in diagnosis and especially in therapy is the number of binding sites of an antibody on its target structure or cell as well as its internalisation rate. These qualities influence the effects and suitability of an antibody for its use especially in therapy.

On the other hand, effector mechanisms relevant for therapeutic use of an antibody are several fold whereby some of the antibodies act via only one mechanism and others by a combination of various effector mechanisms. One strategy is to couple antibodies or antibody fragments to effector molecules which mediate a therapeutic effect, such as coupling to an immune effector molecule, for example interleukine 2 to recruit the immune system, or coupling to toxins or radioisotopes in order to kill target cells, for example for anti-tumor therapies. Radiolabelled antibodies or antibody fragments are also valuable for in vivo diagnosis. The other strategy is to use non-labelled, so-called "naked" antibodies which can have important advantages such as lower toxicology, less complicated logistics, the use of natural immune effector arms or triggering of therapeutic effects without the need of additional effector molecules. A large number of studies have been performed to elucidate the activity mechanisms and mode of action of antibodies as well as develop antibodies using these activities. Among the most important activities and effects are antibody-dependent cell-mediated cytotoxicity activity (herein referred to as "ADCC activity"), the complement-dependent cytotoxicity activity (herein referred to as "CDC activity"), the phagocytosis mediated cytotoxicity activity (herein referred to as "phagocytosis activity"), a receptor mediated activity (herein referred to as "receptor mediated activity") which comprises a whole set of different effects and activities. Receptor mediated activities are mainly based on the binding of the antibody to a molecule or receptor on a target cell or by the prevention of binding of certain molecules to a target cell thereby inducing or inhibiting certain effects on these cells, such as antagonistic or agonistic activity or receptor blockade of an antibody, leading for example to the induction or inhibition of apoptosis or proliferation of target cells or to the secretion or inhibition of the secretion of certain molecules in a target cell, or blocking or triggering certain other receptor mediated events such as interactions between molecules and cells or between cells. The ADCC activity, CDC activity and phagocytosis activity are cytotoxic activities which are mediated by the Fc region of the antibody (herein referred to as "Fc part mediated activities") while the receptor mediated activities, affinity, avidity and specificity of the antibody are mainly mediated via the binding region of the antibody comprised in the Fab region.

The Fc part mediated activities are mediated via immunological effector cells such as killer cells, natural killer cells and activated macrophages, or various complement components by binding to effector ligands such as Fc-gammaRI, Fc-gammaRII, Fc-gammaRIII, C1q component of complement, the neonatal Fc receptor (FcRn), etc. The human IgG1 subclass is reported to have the highest ADCC and CDC activity among human IgG class molecules. For IgM CDC activity is a dominant effector mechanism. The ADCC activity and CDC activity involves the binding of the Fc region, the constant region of the antibody, to Fc-receptors such as Fc-gammaRI, Fc-gammaRII, Fc-gammaRIII of the effector cell or complement components such as C1q. Important amino acid residues are in the hinge region and in particular in the second domain of the constant region ("Cgamma2 domain") whereby the carbohydrate chain bound to the asparagines 297 (Asn-297) of Cgamma2 plays an essential role.

From those activities two are assumed to be influenced by the glycosylation of the antibody: It has been shown that the removal of the sugar chain in the Fc part of the antibody results in the disappearance of the CDC and ADCC activity, while a reduction of galactoses in these N-glycans causes a decrease in CDC activity [Boyd et al., Molecular Immunol., 32, 1311, (1995); US6946292]. Furthermore, it is described that expression of antibodies in rat cells results in an increased ADCC activity of certain antibodies expressed therein [Lifely et al., Glycobiology, 1995 Dec;5(8):813-22; WO00/61739] when compared to the same antibody expressed in hamster and rat cells. Both reports assume that changes in the glycosylation cause this differences in antibody ADCC activity, however, this is not clear, Lifely et al. 1995 assumes that bisecting N-acetylglucosamine ("bisecGlcNAc") is responsible for an increased ADCC activity of the antibody, while WO00/61739 assumes that a dramatic decrease of fucose alphal-6 linked to N-acetylglucosamine at the reducing end of N-glycans ("core-fucose") is responsible for an increased ADCC activity. Furthermore, it is described that recombinant expression of the enzyme GnTIII in CHO cells which is responsible for attaching the sugar bisecGlcNAc to N-glycans results in an increase of the ADCC activity of certain antibodies when expressed in these cells when compared to the same antibody expressed in CHO cells not recombinantly transfected with GnTIII [US 6602684, Umana et al., Nature Biotechn 17: 176-180 (1999)]. Additionally, it was described that the knock-out of the gene FUT8 in CHO cells which results in the lack of core-fucose can increase the ADCC activity of certain antibodies when expressed in such FUT8 KO CHO cells [US 6946292].

Current expression and production systems for antibodies are mainly cell lines derived from rodents and are based on cell lines from hamster, mice, or rat such as CHO, BHK, NS0, Sp2/0 and YB2/0. It is known that rodent cells can produce under non-optimal conditions a number of abnormally glycosylated protein products that lack potency or are immunogenic. In addition, rodent cells are known to express carbohydrate structures with important differences to those expressed in human cells comprising the presence of non-human sugars and the lack of certain human sugar moieties which can render proteins expressed in these cells for example immunogenic, less effective, lower yield or suboptimal structural requirements or folding. Most rodent cells express for example N-glycolylneuraminic acid ("NeuGc") an alternative for N-acetylneuraminic acid ("NeuNAc") not present in humans which is immunogenic in humans and/or the immunogenic galactose alpha(1-3) galactose modification ("Gal alphal-3Gal"), and/or they lack important carbohydrates such as the important alpha2-6 linked NeuNAc or the bisecGlcNAc, as well as other known and unknown differences. In addition, it is known from rodent production that the glycoform profiles are mostly heterogeneous and present also a clone-specific glycoform profile which can vary widely from clone to clone in CHO, NSO, or Sp2/0 cells and are dependent on the mode of production and culture conditions.

From these facts it becomes clear that there is a need for expression and production systems which can further optimise the productivity, homogeneity, and/or antibody activity. Furthermore, from these facts it can not be said which carbohydrate structures and especially which human carbohydrate structures are optimal to improve the activity or homogeneity of antibodies and how a cell line and especially a human cell line has to glycosylate in order to optimise the activity of an antibody.

The present invention provides solutions to these problems by providing new expression systems based on human cells of myeloid leukaemia origin, which surprisingly improve the production of antibodies in respect to activity, yield and/or homogeneity.

This is surprising since so far it was not shown that human myeloid leukaemia cells are suitable for the production of antibodies. While a certain rat leukaemia cell was used for expression of antibodies the glycosylation machinery between human and rat are critically different whereby the latter can express carbohydrate moieties which can be immunogenic in human such as NeuGc and Gal alphal-3 Gal. The rat YB2/0 leukaemia cell was described to yield antibodies with higher ADCC activity due to higher presence of bisecGlcNAc or due to drastic downregulation of core-fucose.

Therefore it was even more surprising that the problem could be solved by this invention using human myeloid cells since it was reported that K562 does not express the enzyme for bisecG1cNAc which was described and shown by gene hybridisation of GnTIII [Yoshimura M et al., Cancer Res. 56(2): 412-8 (1996)] and does express the gene FUT8 which expresses the fucosyltransferase which causes addition of core-fucose as shown by RT-PCR [example 1]. It was surprising because K562 is not resistant to lectin treatment since it is bound strongly by the lectin LCA, the basis for cells negative or strongly down-regulated for core-fucosylation. Hence it could not be expected that those cells can improve the activity of antibodies expressed therein.

It was also surprising that antibodies with increased binding activity, improved homogeneity and/or higher yields can be generated and achieved with the invention than with cells of current expression systems.

The strategy of the invention is to generate suitable human cell lines in order to achieve a system which can provide protein products with a whole set of posttranslational modifications as near as possible to the human system and hence non- or less immunogenic properties.

Due to the facts that carbohydrate structures are very complex, that the glycosylation machinery of cells comprise several hundred enzymes which are involved in their synthesis and that those enzymes are mainly species specific and tissue specific, the major strategy of the inventors to achieve a human glycosylation is to provide a suitable biotechnologically favourable human expression systems favourable to express antibodies. Due to the complexity of the glycosylation machinery and the complexity of carbohydrate structures as well as the fact that glycosylation structures can not be precisely predicted for it is not predictable which glycosylation structures favourable for antibody production will be made by certain human cell types or cell lines. Furthermore no general suitable high expression vector system is known for high yield antibody expression in human cell lines since human cells normally do not lack the DHFR gene and hence do not allow the use of the dhfr/methotrexate amplification system.

INVENTION:

The invention provides biotechnologically favourable methods for the production of antibody molecule compositions having increased activity and/or increased yield and/or improved homogeneity and fully human glycosylation. The invention further provides biotechnologically favourable methods for the production of protein molecule compositions having increased activity and/or increased yield and/or improved homogeneity and fully human glycosylation. It further provides novel host cells, nucleic acids, and molecule compositions.

The invention provides a method for producing an antibody molecule composition comprising:

(a) introducing in a host cell of human myeloid leukaemia origin at least one nucleic acid encoding an antibody molecule or at least one part thereof, and at least one nucleic acid comprising at least one nucleic acid sequence encoding at least one polypeptide of the group of sequence #1 to sequence #9, preferably sequence #1; and
(b) amplifying the nucleic acid sequence encoding said antibody molecule or at least one part thereof by culturing said host cell with methotrexate, preferably by culturing said host cell with at least two successive rounds of methotrexate whereby the concentration of methotrexate is preferably increased by at least about 50%, more preferably by at least about 100%, in each successive round; and
(c) culturing said host cell under conditions which permits the production of said antibody molecule composition, and
(d) isolating said antibody molecule composition.

The invention provides a method for producing an antibody molecule composition having increased activity and/or increased yield and/or improved homogeneity comprising:

(a) introducing in a host cell of human myeloid leukaemia origin at least one nucleic acid encoding an antibody molecule or at least one part thereof; and
(b) culturing said host cell under conditions which permits the production of said antibody molecule composition; and
(c) isolating said antibody molecule composition having increased activity and/or increased yield and/or improved homogeneity.

Furthermore, the invention provides a method for producing an antibody molecule composition having increased activity and/or increased yield and/or improved homogeneity comprising:

(a) introducing in a host cell of human myeloid leukaemia origin at least one nucleic acid encoding an antibody molecule or at least one part thereof, and at least one nucleic acid comprising at least one nucleic acid sequence encoding at least one polypeptide of the group of sequence #1 to sequence #9, preferably sequence #1; and
(b) amplifying the nucleic acid sequence encoding said antibody molecule or at least one part thereof by culturing said host cell with methotrexate, preferably by culturing said host cell with at least two successive rounds of methotrexate whereby the concentration of methotrexate is preferably increased by at least about 50%, more preferably by at least about 100%, in each successive round; and
(c) culturing said host cell under conditions which permits the production of said antibody molecule composition; and
(d) isolating said antibody molecule composition having increased activity and/or increased yield and/or improved homogeneity

Said nucleic acid encoding an antibody molecule or at least one part of it and said nucleic acid comprising at least one nucleic acid sequence encoding at least one polypeptide of the group of sequence #1 to sequence #9, preferably sequence #1, can be one nucleic acid or two separate nucleic acids.

In accordance with the present invention the term "antibody molecule" means any whole antibody or antibody fragment or a molecule comprising an antibody fragment.

Said whole antibody can be any antibody or immunoglobulin molecule of any class or subclass or any molecule comprising at least one immunoglobulin domain known to those skilled in the art comprising but not limited to IgG, IgG1, IgG2, IgG3, IgG4, IgM, IgA, IgD of animal origin such as but not limited to human, simian, rodent, mouse, rat, hamster, rabbit, camel, avian, chicken, or shark origin, and can also be a molecule which comprises protein sequences from antibodies originating from various animals such as chimaeric or humanized antibodies where various percentages of for example murine and human sequences are combined to whole antibodies and /or are mutated for example to decrease immunogenicity or increase affinity as known to those skilled in the art. In another embodiment of the invention said whole antibody can also be the afore described whole antibody with at least one additional amino acid or polypeptide sequence.

In a preferred embodiment said whole antibody is a human, humanized or chimaeric IgG, IgG1, IgG2, IgG3, IgG4, or IgM which comprises a human Fc region. In an even further preferred embodiment of the invention said whole antibody is a human, humanized or chimaeric IgG1, IgG4, or IgM with a human Fc region. Said human Fc region comprises at least one 20 amino acid sequence, more preferably at least 100 amino acids of a constant domain of the Fc region of a human antibody, preferably it comprises at least one Fc domain of a human antibody, more preferably it comprises the human Cgamma2 domain, and more preferably it comprises all constant domains of the Fc region of a human antibody of a certain class or subclass. Said human Fc region can also comprise human sequences from which at least one amino acid was mutated.

In the most preferred embodiment of the invention the whole antibody molecule is either a (i) fully human antibody generated for example from a human antibody producing blood cell or cells or from a transgenic mouse in which the mouse antibody gene locus is at least partially exchanged by human antibody sequences, or (ii) a humanized whole antibody in which at least parts of the variable regions of a murine or rat antibody, such as the framework regions or at least one amino acid of a framework, were exchanged to human sequences or mutated to be less immunogenic in humans which comprise human constant domains, or (iii) a chimaeric whole antibody in which the variable region is murine or rat and comprises human constant domains.

Said fully human antibodies, humanized whole antibodies, and chimaeric whole antibodies or parts thereof as well as the methods to construct, identify, test, optimise, and select these antibody molecules with or without suitable additional sequences as well as methods to construct, identify, test and select most suitable nucleic acids encoding these antibody molecules are known to those skilled in the art.

Said antibody fragment is any fragment of an antibody comprising at least 20 amino acids from said whole antibody, preferably at least 100 amino acids. In a preferred embodiment the antibody fragment comprises the binding region of the antibody such as a Fab, a F(ab)2, multibodies comprising multiple binding domains such as diabodies, triabodies or tetrabodies, single domain antibody or affibodies. In another preferred embodiment the antibody fragment comprises the Fc region with all or parts of its constant domains, preferably comprising the second domain (Cgamma2 domain). In another embodiment the antibody fragment is a truncated whole antibody where at least one amino acid, polypeptide stretches or whole domains are deleted. Those molecules can be combined with additional sequences for stabilization or for improving the binding of the molecules such as linkers.

Said molecule comprising an antibody fragment is any molecule which comprises any of said antibody fragments or other immunoglobulin domains of at least 20 amino acids. In a preferred embodiment said molecule comprising an antibody fragment are fusion molecules where an antibody fragment is fused to other protein sequences such as effector sequences, for example cytokines, co-stimulatory factors, toxins, or antibody fragments from other antibodies in order to generate molecules with multiple binding specificities such as bi- or tri-specific antibodies, or multimerisation sequences such as MBP (mannan binding protein) domains for resulting in the multimerisation of binding domains, or sequences for detection, purification, secretion or stabilization such as tags, localization signals or linkers, or the like. In another preferred embodiment said molecule comprising an antibody fragment are fusion molecules comprising the Fc region of a whole antibody or parts thereof, preferably comprising the second constant domain (Cγ2 domain). Said fusion molecules are fused by genetic means, where the molecules are encoded by one nucleic acid or are fused by co-expression of at least two nucleic acids whereby the fusion is caused by non-covalent or covalent protein interactions or are fused by a combination of both. The genetic fusion between an antibody fragment and another polypeptide sequence or protein molecule can be achieved by genetic engineering where both parts are encoded by a single nucleic acid with or without additional amino acids in between. In a further preferred embodiment said fusion molecules comprise at least one binding region from an antibody fragment such as a single domain antibody, or Fab or a binding sequence not derived from antibodies, such as a lectin domain, and a Fc region or parts thereof comprising the second domain (Cγ2 domain). In another further preferred embodiment the fusion molecules comprise IL-2, IL-12, IL-15, GM-CSF, a peptide toxin, or parts thereof. In another further preferred embodiment said fusion protein comprises at least one binding region from an antibody fragment such as a single domain antibody, Fab or Fab which is linked to multimerisation sequence of MBP.

All those antibody molecules or parts thereof as well as the methods to construct, identify, test and select these antibody molecules with or without suitable additional sequences as well as methods to construct, identify, test and select most suitable nucleic acids encoding these antibody molecules are known to those skilled in the art.

In accordance with the present invention the term "antibody molecule composition" means the molecules of any antibody molecule expressed in a host cell of the invention which can be isolated. Said antibody molecule composition comprises at least one antibody molecule. Said antibody composition comprises at least one glycoform of an antibody molecule. Said glycoform of an antibody molecule means an antibody molecule which carries a particular glycosylation or carbohydrate chain which is different in at least one sugar building block, for example but not limited to an additional galactose, sialic acid, bisecGlcNAc, fucose, or another sugar modification such as but not limited to acetylation or sulfatation from another glycoform of the same antibody molecule. In another embodiment of the invention the antibody molecule composition can comprise molecules of more than one antibody molecule expressed in a host cell. In a preferred embodiment the antibody molecule composition of the invention comprises more molecules in percent of such glycoform or such glycoforms of the antibody molecule which mediate a higher Fc-mediated cellular cytotoxicity and/or an improved binding than an antibody molecule composition of the same antibody molecule obtained from at least one of the cell lines CHO, or CHOdhfr-, or BHK, or NS0, or SP2/0, or PerC.6 or mouse hybridoma, preferably CHOdhfr- [ATCC No. CRL-9096], when expressed therein. In a further preferred embodiment the antibody molecule composition of the invention comprises more molecules of such glycoform or glycoforms of the antibody molecule in percent which mediate a higher Fc-mediated cellular cytotoxicity and/or an improved binding than the antibody molecule composition of the same antibody molecule obtained from the cell lines CHO, or CHOdhfr-, or BHK, or NS0, or SP2/0, or PerC.6 or mouse hybridoma, preferably CHOdhfr- [ATCC No. CRL-9096], when expressed therein.

In accordance with the present invention the term "host cell of human myeloid leukaemia origin" means any cell or cell line of human myeloid leukaemia origin, or any human myeloid or myeloid precursor cell or cell line which can be obtained from a leukaemia patient, or any myeloid or myeloid precursor cell or cell line which can be obtained from a human donor, or a cell or cell line derived from anyone of said host cells, or a mixture of cells or cell lines comprising at least one of those aforementioned cells.

In another embodiment of the invention said host cell of human myeloid leukaemia origin of the invention also comprise such cells or cell lines which were obtained by fusing at least one of aforementioned host cells of myeloid leukaemia origin with another cell of human or animal origin, such as but not limited to B cells, CHO cells. Those skilled in the art are able to identify and use suitable sources and methods to obtain, generate and/or immortalize suitable cells and cell lines from humans for suitable host cells of human myeloid leukaemia origin.

The term cell or cell line derived from said host cell means any cell or cell line which can be obtained by any means of cultivation and cloning with or without prior mutation or genetic engineering of said host cell of myeloid leukaemia origin and comprises selection of those cells or cell lines derived from said host cell with the desired properties. Said cultivation and cloning is based on the fact that cell clones with differing properties can be obtained from primary cell cultures, cultures of cells and even cultures of cell clones by multiple rounds of passaging and cloning of the cells using preferably single cell cloning methods such as limited dilution or flowcytometry based cell sorting. In a preferred embodiment said cell or cell line derived from said host cells is selected by binding to a lectin or carbohydrate-binding antibody. Said mutation can be performed by treatment known to those skilled in the art with physical, chemical or biological mutagens, such as but not limited to radiation, alkylating agents or EMS (ethylmethanesulfonate), proteins such as lectins, or virus particles. Said genetic engineering can be performed by methods known to those skilled in the art such as knock-out of genes via site specific homologous recombination, use of transposons, site-specific mutagenesis, transfection of certain nucleic acids, or silencing of genes, or gene products. Methods for said cultivation and cloning, said mutation and mutagens and said genetic engineering are known to those skilled in the art and some examples are described in detail in WO2005/017130 A2, US 2003/0115614 A1, or are described herein. Those skilled in the art are able to select and/or adopt and/or modify a suitable method or combination of methods for generation of a suitable cell or cell line derived from said host cell of the invention.

Said cell or cell lines derived from said host cell are selected due to properties of those cells which are advantageous when compared to their parent cell or cell line such as but not limited to shorter doubling times, faster growth, possibility to grow under higher densities, can produce more, are growing under serum free conditions and/or in protein free media, higher cloning efficiencies, higher transfection efficiencies for DNA, higher expression rates of antibody molecule compositions, higher activities for an antibody molecule composition expressed therein, higher homogeneities of an antibody molecule composition expressed herein, and/or higher robustness to scaling up. Methods for selecting those cell with advantageous properties are known to those skilled in the art or described herein.The invention provides a method for generating a host cell of the invention, comprising (a) incubating a human myeloid leukaemia cell with a lectin or an antibody recognizing a desialylated epitope or an epitope lacking a sialic acid but which binds not or significantly less the sialylated form of the epitope, and (b) isolating cells bound to said lectin or antibody, and (c) culturing the isolated cells for a suitable time, and (d) select a cell, cells or a cell clone which strongly binds to a lectin or an antibody which binds to an epitope with sialic acid. More preferred is the method as described above, wherein said lectin or said antibody recognizing a desialylated epitope or an epitope lacking a sialic acid is Nemod-TF1, Nemod-TF2, A78-G/A7, or PNA and wherein said human myeloid leukaemia cell is the cell line K562, KG-1, MUTZ-3, NM-F9 [DSM ACC2606], NM-D4 [DSM ACC2605], NM-H9, H9, NM-H10.

The method for generating a host cell of the invention with high sialylation and favourable biotechnological properties such as rapid cell growth comprising (i) incubating a human myeloid leukaemia cell of the invention as originator cell, preferably K562, with a lectin or preferably an antibody recognizing a desialylated epitope or an epitope lacking a sialic acid but which binds not or less the sialylated form of the epitope, such as but not limited to Nemod-TF1, Nemod-TF2, A78-G/A7, or PNA (lectin from Arachis hypogaea, peanut agglutinin), preferably bound to magnetic beads, and (ii) isolating cells bound to said lectin or antibody, and (iii) culturing the isolated cells for a suitable time, and (iv) select a cell, cells or a cell clone, preferably after single cell cloning, which strongly binds to a lectin or an antibody which binds to an epitope with sialic acid, such as SNA (Sambucus nigra agglutinin) or MAL (Maackia amurensis lectin), MAL I (Maackia amurensis lectin I), preferably SNA.

In a preferred embodiment the invention provides a method for generating a host cell of the invention with high sialylation and favourable biotechnological properties such as rapid cell growth comprising (i) incubating K562 with Nemod-TF1, Nemod-TF2, A78-G/A7, or PNA bound to magnetic beads, and (ii) isolating cells bound to said lectin or antibody, and (iii) culturing the isolated cells for a suitable time of about one to 6 weeks, and (iv) select a cell clone after single cell cloning, which strongly binds to SNA. In a preferred embodiment those cell bind stronger to SNA than the originator cell, and in another preferred embodiment they grow faster than the originator cell.

In a preferred embodiment of the invention the originator cell is treated with ethylmethanesulfonate prior to step (i).

Those skilled in the art are able to select suitable conditions and methods and optimise them in order to generate these host cells in sense of the invention. More details for some of the steps can be found under WO2005/017130 A2 and WO2005/080585 A1.

In a preferred embodiment, said preferred cell line generated is H9, preferably NM-H9, more preferably NM-H10, most preferably NM-H9D8.

As the most preferred cell clone generated by the method described above, NM-H9D8 was selected and deposited under DSM ACC_____ at the "DSMZ-Deutsche Sammlung von Mikroorganismen und Zellkulturen GmbH" in Braunschweig (Germany), by Glycotope GmbH, Robert-Rössle-Str. 10, 13125 Berlin (Germany) at the September 15, 2006.

Other cell clones as NM-E-2F9, NM-C-2F5, or NM-H9D8-E6 were selected by incubation of the parental cells with one or more lectins and following single cell cloning using flow cytometry cell sorting whereby a positive selection procedure or a combination of negative and positive selection was performed. To get cell clones with stable characteristics obtained cell clones were recloned at least once by limited dilution as described above.

In a preferred embodiment of the invention said host cell of myeloid leukaemia origin grow and produce an antibody molecule composition of the invention under serum-free conditions. In an even further preferred embodiment said host cell of myeloid leukaemia origin grow under serum-free conditions and nucleic acid encoding the antibody molecule can be introduced in these cells and an antibody molecule composition is isolated under serum-free conditions.

In a preferred embodiment of the invention the host cell of human myeloid leukaemia origin of the invention is the cell or cell line K562, KG1, MUTZ-3, NM-F9 [DSM ACC2606], NM-D4 [DSM ACC2605], NM-H9, H9, NM-H10, or a cell or cell line derived from anyone of said host cells, or a mixture of cells or cell lines comprising at least one of those aforementioned cells.

In a further preferred embodiment of the invention the host cell of human myeloid leukaemia origin of the invention is the cell or cell line K562, NM-F9 [DSM ACC2606], NM-D4 [DSM ACC2605], NM-H9, H9, NM-H10, or a cell or cell line derived from anyone of said host cells.

In a preferred embodiment of the invention the host cell of human myeloid leukaemia origin of the invention is the cell or cell line K562, such as K562 [ATCC CCL-243], or a cell or cell line derived from anyone of said host cell.

In a further preferred embodiment of the invention the host cell of human myeloid leukaemia origin of the invention is the cell or cell line K562, NM-F9 [DSM ACC2606], NM-D4 [DSM ACC 2605], NM-H9, H9, NM-H10, NM-E-2F9, NM-C-2F5, NM-H9D8, or NM-H9D8-E6, or a cell or cell line derived from anyone of said host cells.

In a further preferred embodiment of the invention the host cell of human myeloid leukaemia origin of the invention is the cell or cell line K562, NM-F9 [DSM ACC2606], NM-D4 [DSM ACC2605], NM-H9, H9, NM-H10, NM-E-2F9, NM-C-2F5, NM-H9D8, or NM-H9D8-E6.

In a further preferred embodiment of the invention the host cell of human myeloid leukaemia origin of the invention is the cell or cell line NM-F9 [DSM ACC2606], NM-D4 [DSM ACC2605], NM-H9, H9, NM-H10, NM-E-2F9, NM-C-2F5, NM-H9D8, or NM-H9D8-E6.

In an even more preferred embodiment of the invention the host cell of human myeloid leukaemia origin of the invention is the cell, cells or cell line K562, NM-F9 [DSM ACC2606], NM-D4 [DSM ACC2605], NM-H9, H9, NM-H10, NM-E-2F9, NM-C-2F5, NM-H9D8, or NM-H9D8-E6 which grow and produce an antibody molecule composition of the invention under serum-free conditions, and most preferred hereunder cell, cells or cell line growing under serum-free conditions and the nucleic acid encoding the antibody molecule can be introduced in these cells and an antibody molecule composition is isolated under serum-free conditions.

In the most preferred embodiment of the invention the host cell of human myeloid leukaemia origin of the invention is the cell, cells or cell line NM-F9 [DSM ACC2606], NM-D4 [DSM ACC2605], NM-H9, H9, NM-H10, NM-E-2F9, NM-C-2F5, NM-H9D8, or NM-H9D8-E6 which grow and produce an antibody molecule composition of the invention under serum-free conditions, and most preferred hereunder cell, cells or cell line growing under serum-free conditions and the nucleic acid encoding the antibody molecule can be introduced in these cells and an antibody molecule composition is isolated under serum-free conditions.

According to the invention the term introducing a nucleic acid means any method known to those skilled in the art to introduce a nucleic acid, or two or more nucleic acids into a mammalian host cell or cells by methods such as but not limited to electroporation, transfection using cationic lipids, calcium phosphate, DEAE-dextran, or infection by virus particles such as adenoviruses or retroviruses or a combination thereof.

Preferred embodiments are described in methods.

Those skilled in the art are able to select and optimise a suitable method for introduction of one or more nucleic acids of the invention.

In accordance with the present invention the nucleic acid encoding an antibody molecule is any nucleic acid which encodes the antibody molecule or at least one part thereof. The antibody molecule of the invention can thereby be encoded by a single or by multiple nucleic acid molecules. Said part of an antibody molecule encoded by said nucleic acid comprises at least one 20 amino acid sequence, more preferably at least 100 amino acids of an antibody molecule or of a constant and/or variable domain of the antibody molecule. The comprised sequence encoding the antibody molecule or at least one part thereof can be separated by at least one other sequence, such as an intron. The sequence of a domain can comprise at least one amino acid mutation.

In a preferred embodiment the nucleic acid encoding an antibody molecule or at least one part of it comprises at least one variable and/or at least one constant domain of the antibody molecule, more preferably both, more preferably such which comprises a human sequence at least in the part of the constant domain or domains, and even more preferred such which comprises the human Cgamma2 domain, and most preferably it comprises all constant domains of the Fc region of a human antibody of a certain class or subclass and the variable domain.

In a preferred embodiment the antibody molecule is encoded by a single nucleic acid.

In another preferred embodiment the antibody molecule is encoded by two nucleic acids or by three nucleic acids.

In a further preferred embodiment one nucleic acid encodes one part of the antibody molecule which encodes for the variable and/or the constant domain of the light chain and another nucleic acid encodes for another part of the antibody molecule which encodes for the variable and/or at least one constant domain of the heavy chain.

In accordance with the present invention the nucleic acid comprising at least one nucleic acid sequence encoding at least one polypeptide of the group of sequence #1 to sequence #9 means that said nucleic acid encodes for at least one polypeptide of the group of sequence #1 to sequence #9, preferably sequence #1. Any number of these sequences is suitable, as long as it can be introduced successfully into the host cell of the invention. In a preferred embodiment said nucleic acid encodes for one, two or three polypeptides of the group of sequence #1 to sequence #9, more preferably for one polypeptide, and most preferably for the polypeptide of sequence #1. In sense of the invention said nucleic acid can also encode for a polypeptide of the group of sequence #1 to sequence #9, preferably sequence #1, which has at least one amino acid mutation as long as this mutation allows the amplification of the nucleic acid encoding an antibody molecule or at least one part thereof by methotrexate as described elsewhere herein.

The nucleic acid sequence encoding at least one polypeptide of the group of sequence #1 to sequence #9, preferably sequence #1, can be part of the same nucleic acid molecule as the nucleic acid encoding an antibody molecule or at least one part thereof as described elsewhere herein or on separate nucleic acid molecules.

In another preferred embodiment of the invention a separate nucleic acid comprising a nucleic acid sequence encoding a sequence selected from the group of sequence #1 to sequence #9, most preferably sequence #1, can be introduced into the host cell of the invention separately from said nucleic acid or nucleic acids encoding the antibody molecule or parts thereof of the invention. This can be done either by introducing said separate nucleic acid comprising a nucleic acid sequence encoding a sequence selected from the group of sequence #1 to sequence #9, together, before or after introducing said nucleic acid or nucleic acids encoding the antibody molecule or parts thereof of the invention. In a preferred embodiment this is performed in parallel and in another preferred embodiment the host cell of the invention already comprises said separate nucleic acid comprising a nucleic acid sequence encoding a sequence selected from the group of sequence #1 to sequence #9, most preferably sequence #1.

Further preferred embodiments are described in the examples.

Amplification of stably introduced said nucleic acid or several copies of said nucleic acid encoding the antibody molecule or fraction thereof can be performed as described elsewhere herein and in the examples using methotrexate.

In a preferred embodiment the nucleic acid encoding an antibody molecule or at least one part thereof comprise at least one other genetic element which allow the selection of those cells in which the nucleic acid was successfully introduced, such as but not limited to antibiotic resistance genes, such as but not limited to the genetic element coding for a puromycin or neomycin resistance. Furthermore these nucleic acids comprise one or several genetic elements such as promoters, enhancers, polyadenylation sites, and/or introns, for expression of the antibody molecule in the host cells of the invention, and genetic elements such as bacterial origin of replication, promoters and elements for selection of transfected bacteria such as antibiotic resistance genes for multiplying the nucleic acid in a bacteria.

Suitable promoters include the promoter of IE (immediate early) gene of cytomegalovirus (CMV), SV40 early promoter, the promoter of a retrovirus, metallothionein promoter, heat shock promoter, SR alpha promoter, EF-1 alpha promoter, etc. The enhancer of IE gene of human CMV may be used in combination with the promoter.

Those and further genetic elements are known to those skilled in the art and can be selected, combined, optimised and introduced into said nucleic acid encoding the antibody molecule or at least one part thereof by those skilled in the art. Preferred embodiments of said nucleic acid encoding an antibody molecule or at least one part thereof or a combination of nucleic acids are described herein and in the examples as well as preferred genetic elements for use and combination, however, the invention is not restricted to the use of those and can be combined or further optimised by those skilled in the art.

Those skilled in the art are able to select and/or combine the suitable genetic element, construct the according nucleic acid vectors or elements to introduce one or more nucleic acids of the invention into a cell line according to the invention.

Said nucleic acid or combination of nucleic acids encoding the antibody molecule or at least one part thereof, as well as said additional genetic elements, as well as the methods to introduce them such as transfecting them in the host cells of the invention for expression of the antibody molecule composition or into bacteria for multiplication are known to those skilled in the art as well as the methods to construct, identify, test, optimise, select and combine these nucleic acid or acids and combine them with suitable additional sequences for selection of those cells which are successfully transfected as well as methods to construct, identify, test and select most suitable nucleic acids encoding these antibody molecules are known to those skilled in the art.

Preferred embodiments of the invention are described in detail in the examples.

In a preferred embodiment of the invention the nucleic acid encoding an antibody molecule or at least one part thereof comprises a genetic element for selection of those host cells of the invention in which the nucleic acid is successfully introduced such as but not limited to neomycin or puromycin, more preferably it comprises in addition a promoter such as EF-1alpha or CMV promoter, more preferably it comprises in addition a CMV enhancer, more preferably it comprises in addition a genetic element which allows the selection of bacteria which are transfected with the nucleic acid and a genetic element for replication such as the ColE1 replication origin for multiplication of said nucleic acid in bacteria, and even more preferably it comprises in addition a genetic element for multiplication of said the nucleic acid in COS cells such as the SV40 origin. These elements are known to those skilled in the art and can be selected, combined, optimised and used by those skilled in the art.

In an even further preferred embodiment of the invention said above described nucleic acid encoding an antibody molecule or at least one part thereof comprises at least one sequence encoding the variable domain and at least one constant domain of the heavy and/or the light chain of the antibody molecule.

In an even further preferred embodiment of the invention said above described nucleic acid encoding an antibody molecule or at least one part thereof comprises at least one sequence encoding the variable domain and constant domain of the light chain of the antibody molecule or the variable domain and all constant domains of the heavy chain of the whole antibody molecule.

In an even further preferred embodiment of the invention one of said above described nucleic acid encodes at least one part of the antibody molecule comprising at least one sequence encoding the variable domain and the constant domain of the light chain of the antibody molecule and a second of said above described nucleic acid encodes at least one other part of the antibody molecule comprising at least one sequence encoding the variable domain and at least one constant domain, preferably all constant domains of the heavy chain of the antibody molecule, and both nucleic acids are introduced into the same host cell of the invention. Preferably one of said nucleic acids encodes additionally for the genetic element encoding puromycin resistance and the other said nucleic acid encodes additionally for the genetic element encoding neomycin resistance.

In an even more preferred embodiment one of said nucleic acids encodes additionally for the genetic element encoding a puromycin or neomycin resistance and the other said nucleic acid comprises in addition a nucleic acid sequence encoding at least one sequence selected from the group of sequence #1 to sequence #9, most preferably sequence #1. In a even more preferred embodiment one of said nucleic acids comprising sequences encoding the variable domain and the constant domain of the light chain and comprises in addition a nucleic acid sequence encoding at least one sequence selected from the group of sequence #1 to sequence #9, most preferably sequence #1, and the other said nucleic acid comprises sequences encoding the variable domain and at least one constant domain, preferably all constant domains, of the heavy chain of the antibody molecule and comprises in addition a nucleic acid sequence encoding for a puromycin or neomycin resistance, preferably puromycin resistance.

In an even further preferred embodiment of the invention said nucleic acid encoding the antibody molecule or at least one part thereof comprises at least one sequence encoding the variable domain and the constant domain of the light chain of the antibody molecule and at least one sequence encoding the variable domain and at least one constant domain, preferably all constant domains of the heavy chain of the antibody molecule.

In a preferred embodiment of the invention the constant domains encoded by above or elsewhere described nucleic acids of the invention are human constant domains of IgG or IgM, preferably, human IgG1, IgG4 or IgM, or one domain or a combination of domains thereof, whereby preferably genomic sequences or sequences derived from genomic sequences comprising at least one intron are used, which can be selected, constructed and optimised by those skilled in the art.

In a further preferred embodiment of the invention said nucleic acid encoding the antibody molecule or at least one part thereof of the invention further comprises the EF-1alpha promoter / CMV enhancer or a CMV promoter derived promoter, preferably CMV-E.

In a further preferred embodiment of the invention said nucleic acid encoding the antibody molecule or at least one part thereof of the invention further comprises a secretion signal peptide, preferably the T cell receptor secretion signal.

In an even further preferred embodiment of the invention said nucleic acid encoding the antibody molecule or at least one part thereof of the invention further comprises a secretion signal peptide, preferably the sequence #10.

Said nucleic acid or combination of nucleic acids encoding the antibody molecule or at least one part thereof, as well as said additional genetic elements, as well as the methods to introduce them are known to those skilled in the art as well as the methods to construct, identify, test, optimise, select and combine these nucleic acid or acids and combine them with suitable additional sequences for selection of those cells which are successfully transfected as well as methods to construct, identify, test and select most suitable nucleic acids encoding these antibody molecules are known to those skilled in the art.

Preferred embodiments of the invention are described in detail in the examples.

The introduction of the nucleic acid can be either transient or stable.

In accordance with the present invention the term amplifying the nucleic acid sequence encoding an antibody molecule or at least one part thereof by culturing said host cell with methotrexate means that a host cell of the invention described elsewhere herein in which at least one nucleic acid encoding an antibody molecule or at least one part thereof, and at least one nucleic acid comprising at least one nucleic acid sequence encoding at least one polypeptide of the group of sequence #1 to sequence #9, preferably sequence #1, was introduced is cultivated by at least one methotrexate concentration. A typical cultivation time is between one to two weeks for each round, at typical concentrations from about 20 nM bis 3000 nM, preferably between about 50 nM to 2000 nM, more preferably between about 100 nM to 2000 nM. The duration of a methotrexate amplification treatment as well as the concentration and the according vectors of nucleic acids of the invention can be optimised by those skilled in the art and is also described in a preferred embodiment in the examples. Optimal amplification conditions can differ between different antibody molecules encoded and usage of different nucleic acids or nucleic acid constructs or combinations thereof described afore. Those skilled in the art are able to select and optimise the most suitable conditions and nucleic acids of the invention. Said amplification leads to an integration of more nucleic acid copies encoding the antibody molecule or at least one part thereof into the genome of the host cell than without methotrexate cultivation or than without the introduction of the nucleic acid sequence encoding at least one polypeptide of the group of sequence #1 to sequence #9 comprising and/or it leads to an increased production of the antibody molecule composition.

In a preferred embodiment of the invention said host cell for amplification of the nucleic acid or nucleic acids encoding an antibody molecule or at least one part thereof, which was introduced in said host cell in which at least one nucleic acid comprising at least one nucleic acid sequence encoding at least one polypeptide of the group of sequence #1 to sequence #9, preferably sequence #1, was introduced, as described elsewhere herein including its preferred embodiments, are a human myeloid leukaemia origin, preferably the cell or cell line KG1, MUTZ-3, K562, NM-F9 [DSM ACC2606], NM-D4 [DSM ACC2605], NM-H9, H9, NM-H10, NM-E-2F9, NM-C-2F5, NM-H9D8, or NM-H9D8-E6, or a cell or cell line derived from anyone of said host cells, more preferably the cell or cell line K562, NM-F9 [DSM ACC2606], NM-D4 [DSM ACC2605], NM-H9, H9, NM-H10, NM-E-2F9, NM-C-2F5, NM-H9D8, or NM-H9D8-E6, or a cell or cell line derived from anyone of said host cells, and even more preferably the cell or cell line NM-F9 [DSM ACC2606], NM-D4 [DSM ACC2605], NM-H9, H9, NM-H10, NM-E-2F9, NM-C-2F5, NM-H9D8, or NM-H9D8-E6, or a cell or cell line derived from anyone of said host cells.

In a preferred embodiment of the invention said host cell is cultivated with at least two successive rounds of methotrexate whereby the concentration of methotrexate is preferably increased by at least about 50%, more preferably by at least about 100%, in each successive round. In an even further preferred embodiment of the invention said host cell is cultivated with at least three, more preferably with four, more preferably with 5, and even more preferably with 6 successive rounds of methotrexate, whereby the concentration of methotrexate is preferably increased by at least about 50%, more preferably by at least about 100%, in each successive round. Even more preferred are concentrations of methotrexate between about 20 nM and 3000 nM, more preferred between about 50 nM to 2000 nM, more preferably between about 100 nM to 2000 nM, and even more preferred of about 100 nM, 200 nM, 500 nM, 1000 nM, 2000 nM which are in the preferred embodiment are used in successive rounds starting from 100 nM.

It is surprising that the introduction of a nucleic acid sequence encoding at least one polypeptide of the group of sequence #1 to sequence #9 allows the amplification of the nucleic acid sequence encoding the said antibody molecule or at least one part thereof in the host cell of human myeloid leukaemia origin of the invention, and especially in K562, NM-F9 [DSM ACC2606], NM-D4 [DSM ACC2605], NM-H9, H9, NM-H10, NM-E-2F9, NM-C-2F5, NM-H9D8, or NM-H9D8-E6, or a cell or cell line derived from anyone of said host cells, by culturing with methotrexate.

It is especially surprising that the introduction of a nucleic acid sequence encoding at least one polypeptide of sequence #1 allows the amplification of the nucleic acid sequence encoding the said antibody molecule or at least one part thereof in the host cell of the invention, and especially in K562, NM-F9 [DSM ACC2606], NM-D4 [DSM ACC2605], NM-H9, H9, NM-H10, NM-E-2F9, NM-C-2F5, NM-H9D8, or NM-H9D8-E6, or a cell or cell line derived from anyone of said host cells, by culturing with methotrexate.

It is even more surprising that the introduction of a nucleic acid sequence encoding at least one polypeptide of the group of sequence #1 to sequence #9 allows an even further amplification of the nucleic acid sequence encoding the said antibody molecule or at least one part thereof in the host cell of the invention, and especially in K562, NM-F9 [DSM ACC2606], NM-D4 [DSM ACC2605], NM-H9, H9, NM-H10, NM-E-2F9, NM-C-2F5, NM-H9D8, or NM-H9D8-E6, or a cell or cell line derived from anyone of said host cells, by culturing said host cell with at least two successive rounds of methotrexate whereby the concentration of methotrexate is increased by at least about 50%, more preferably by at least about 100%, in each successive round.

It is even more surprising that the introduction of a nucleic acid sequence encoding at least one polypeptide of sequence #1 allows an even further amplification of the nucleic acid sequence encoding the said antibody molecule or at least one part thereof in the host cell of the invention , and especially in K562, NM-F9 [DSM ACC2606], NM-D4 [DSM ACC2605], NM-H9, H9, NM-H10, NM-E-2F9, NM-C-2F5, NM-H9D8, or NM-H9D8-E6, or a cell or cell line derived from anyone of said host cells, by culturing said host cell with at least two successive rounds of methotrexate whereby the concentration of methotrexate is increased by at least about 50%, more preferably by at least about 100%, in each successive round.

In a preferred embodiment that amplification is stable meaning that it leads to the production of high yields of the antibody molecule composition over at least 35 generations of host cell division cycles.

Amplification of stably introduced said nucleic acid or nucleic acids encoding the antibody molecule or fraction thereof can be performed as described above and in the examples using methotrexate.

Further preferred embodiments are described in the examples.

In a preferred embodiment of the invention the host cell of the invention with introduced nucleic acids of the invention are preferably used after at least one round of single cell cloning and selection of those cell clones with suitable expression and secretion of said antibody composition. Preferably said single cell cloning and selection of those cell clones with suitable expression and secretion of said antibody composition occurs after at least one round of methotrexate amplification described elsewhere herein. In a further preferred embodiment, said cell clones are further amplified by at least one additional round of amplification with methotrexate, preferable an increased concentration of methotrexate, preferably at least about the double concentration of methotrexate, and even more preferred followed by a further round of single cell cloning and selection of those cell clones with suitable expression and secretion of said antibody composition. With these preferred embodiments cell clones with particularly high expression yields can be selected.

In accordance with the present invention the term culturing said host cell under conditions which permits the production of said antibody molecule composition means that the host cell of the invention comprising at least one nucleic acid encoding an antibody molecule, preferably the preferred embodiments of said nucleic acid of the invention described elsewhere herein, is cultured under culture conditions which allow the expression of the antibody molecule in form of the antibody molecule composition, preferably secretion into the medium, preferably with high yields and/or high activity and/or high homogeneity as described elsewhere herein. Those skilled in the art are able to select the most suitable culture conditions by using suitable media and culture conditions such as but not limited to suitable time, temperature, pH, gasing, feed, medium, medium supplements, vessel or reactor sizes and principles known to those skilled in the art. Those skilled in the art are able to select and optimise the most suitable conditions. Preferred embodiments are described in examples but are not limited to those.

Culturing of the cells of the present invention can be carried out by any of general culturing methods for animal cells capable of efficiently producing the desired antibody molecule composition, for example, batch culture, repeated batch culture, fed-batch culture and perfusion culture. Preferably, fed-batch culture or perfusion culture is employed in order to raise the productivity of the desired polypeptides.

In a further preferred embodiment of the invention said culturing is performed under serum-free conditions and even further preferred with protein free media or animal component free media.

Adaptation of host cells of the present invention to a serum-free medium in accordance with the present invention is surprisingly fast and robust. Adaptation can be carried out, for example, by adapting cells subcultured in a serum-containing medium directly to a commercially available serum-free medium, or by continuous adaptation whereby the direct adaptation to serum-free medium is preferred and advantageous. During the process of adaptation to a serum-free medium, the viability of cells lowers temporarily, which sometimes causes extinction of cells. Therefore, it is preferred to inoculate cells into a medium for the adaptation to a serum-free medium at a cell density of $1 \times 10^5$ to $5 \times 10^5$ cells/ml, preferably $2 \times 10^5$ cells/ml, in order to restore the viability of cells or to keep it high. After 4 to 7 days of culturing, the cells whose density reached $5 \times 10^5$ to $10 \times 10^5$ cells/ml are selected as the cells adapted to a serum-free medium.

Adaptation to serum-free medium can be also performed by successive dilution of the medium supplemented with FCS by a serum-free medium composition (continuous adaptation). Those skilled in the art are able to select and optimise the most suitable conditions. Preferred embodiments are described in examples but are not limited to those.

After the cells of the present invention are adapted to a serum-free medium, a cloned cell line can be prepared by using the limiting dilution method with a 96-well plate, the colony forming method, or the like, and cells or cell line are selected due to properties of those cells which are aventageous when compared to their parent cell or cell line such as but not limited to shorter doubling times, faster growth, possibility to grow under higher densities, can produce more, higher cloning efficiencies, higher transfection efficiencies for DNA, higher expression rates of antibody molecule compositions, higher activities for an antibody molecule composition expressed therein, higher homogeneities of an antibody molecule composition expressed herein, and/or higher robustness to scaling up. Methods for selecting those cell with advantageous properties are known to those skilled in the art or described herein.

In accordance with the present invention the term isolating said antibody molecule composition means that the antibody molecule composition expressed by said host cell comprising at least one of said nucleic acids encoding the antibody molecule or fraction thereof described elsewhere herein is gained by using the culture media after culturing or further enriching or purifying the antibody molecule composition or parts of said antibody molecule composition by methods known to those skilled in art. Said antibody molecule composition in sense of the invention also means parts of said antibody molecule composition enriched for certain antibody molecules described elsewhere herein. In one preferred embodiment the antibody molecule composition is isolated by separating the media after culturing from the cells and/or cell debris for example by centrifugation techniques.

In a further preferred embodiment of the invention an antibody molecule composition of the invention is isolated or further enriched by ultrafiltration, precipitation methods or other concentration methods known to those skilled in the art. In a further preferred embodiment of the invention an antibody molecule composition of the invention is isolated by purification of the antibody molecule composition by chromatographic methods such as but not limited to affinity chromatography using according affinity materials such as but not limited to Protein A, Protein G, anti-antibody isotype antibodies, lectin chromatography, antibodies against a certain tag introduced into antibody molecule such as HIS-tag or myc-tag, or antigen, or by ion exchange chromatography known to those skilled in the art.

In a preferred embodiment of the invention an antibody molecule composition of the invention of mainly IgG is isolated by Protein A chromatography with or without prior ultracentrifugation.

In another preferred embodiment of the invention an antibody molecule composition of the invention of mainly IgM is isolated by anti-IgM antibody chromatography with or without prior ultracentrifugation.

In another preferred embodiment of the invention an antibody molecule composition of the invention enriched in certain glycoforms of the antibody molecule is isolated by lectin affinity chromatography with or without prior ultracentrifugation. Further methods of purifying or enriching proteins or certain glycoforms of proteins are known to those skilled in the art and can be selected, adopted, optimised and used alone or in combination with afore described methods by those skilled in the art to isolate or further purify, fractionate or enrich the antibody molecule composition or fractions thereof of the invention.

In a preferred embodiment the antibody molecule composition is isolated using Protein A columns. In another preferred embodiment the antibody molecule composition is isolated using an anti-IgM column.

In accordance with the present invention the term "increased activity" means that the activity of an antibody molecule composition of the invention expressed in a host cell of human myeloid leukaemia origin is higher than the activity of at least one antibody molecule composition from the same antibody molecule when expressed in at least one of the cell lines CHO, or CHOdhfr-, or BHK, or NS0, or SP2/0, or PerC.6 or mouse hybridoma. In the preferred embodiment of the invention it means that the activity of an antibody molecule composition of the invention expressed in a host cell of human myeloid leukaemia origin is higher than the activity of an antibody molecule composition from the same antibody molecule when expressed in CHOdhfr- [ATCC No. CRL-9096] Said increased acitivity is an increased Fc-mediated cellular cytotoxicity or an increased binding activity.

Said increased Fc-mediated cellular cytotoxicity is an increased antibody dependent cellular cytotoxicity (ADCC activity), complement dependent cytotoxicity (CDC activity), and/ or cytotoxicity caused by phagocytosis (phagocytosis activity). The increased Fc-mediated cellular cytotoxicity, including ADCC activity, CDC activity or phagocytosis activity can be determined by various methods known to those skilled in the art and some are described in detail in examples without limiting it to those methods whereby the methods described in the examples are preferred embodiments of the invention.

Said increased binding activity is an increased binding to the epitope of the antibody molecule, such as the epitope, the antigen, another polypeptide comprising the epitope, or a cell comprising the epitope of the antibody molecule, or an increased binding to at least one Fc receptor or another effector ligand, such as Fc-gammaRI, Fc-gammaRII, Fc-gammaRIII, , and subclasses therefrom such as Fc-gammaRIIa, Fc-gammaRIIIa, Fc-gammaRIIIb, or C1q component of complement, or FcRn or a molecule or cell comprising any of those Fc receptors or effector ligands. The increased binding activity can be a higher affinity, a higher avidity, and/or a higher number of binding sites or combinations thereof. The increased binding activity can result in various effects and activities such as but not limited to forms of receptor mediated activity as described in the Background of the art. The increased binding affinity, avidity and receptor mediated activity can be determined by at least one of the various methods known to those skilled in the art such as but not limited to Biacore measurement, Scatchard analysis, ELISA or RIA based measurements, flow cytometry measurements, test for determining the apoptosis induction in suitable target cells, tests for determination of the proliferation of suitable target cells, test for antagonistic, agonistic and/or receptor blockade of an antibody molecule composition such as but not limited to inhibition of cell-cell mediated binding, trigger of cell internal molecular events. Those skilled in the art are able to select and/or adopt and/or modify a suitable method or combination of methods for testing the binding affinity, avidity, number of binding sites and/or receptor mediated activity.

The methods of the invention can be used to test the ability of an antibody to be able to obtain an increased Fc-mediated cellular cytotoxicity, preferably ADCC activity, CDC activity and/ or cytotoxicity caused by phagocytosis, and/or an increased binding activity to the epitope of the antibody molecule or preferably to at least one Fc receptor or another effector ligand, in general and/or in particular with the host cells of the invention and its preferred embodiments described elsewhere herein.

In a preferred embodiment of the invention the activity of an antibody molecule composition of the invention expressed in a host cell of human myeloid leukaemia origin is higher than the activity of at least one antibody molecule composition from the same antibody molecule from at least one of the cell lines CHO, or CHOdhfr-, or BHK, or NS0, or SP2/0, or PerC.6 or mouse hybridoma, preferably CHOdhfr- [ATCC No. CRL-9096], when expressed therein.

In a preferred embodiment of the invention the activity of an antibody molecule composition of the invention expressed in a host cell of human myeloid leukaemia origin is at least 50 % higher than the activity of the antibody molecule composition from the same antibody molecule expressed in the cell line CHOdhfr- [ATCC No. CRL-9096], more preferably at least 2 times, more preferably at least 3 times, more preferably at least 4 times, more preferably at least 5 times, more preferably at least 7 times, more preferably at least 10 times, more preferably at least 15 times, more preferably at least 23 times, more preferably at least 30 times, more preferably at least 50 times, more preferably at least 75 times, more preferably at least 100 times, more preferably at least 150 times, more preferably at least 150 times, more preferably at least 230 times, more preferably at least 300 times, more preferably at least 500 times, more preferably at least 750 times, and most preferably more than 1000 times.

In a preferred embodiment of the invention the increased activity of an antibody molecule composition is an increased ADCC activity. In another preferred embodiment the increased activity of an antibody molecule composition is an increased CDC. In another preferred embodiment the increased activity of an antibody molecule composition is an increased cytotoxicity caused by phagocytosis. In another preferred embodiment the increased activity of an antibody molecule composition is an increased binding activity to the epitope. In another preferred embodiment the increased activity of an antibody molecule composition is an increased binding activity to at least one Fc receptor, preferably FcγRIIIA. In a further preferred embodiment the increased activity of an antibody molecule composition is an increased Fc-mediated cellular cytotoxicity and an increased binding activity to the epitope. In a further preferred embodiment the increased activity of an antibody molecule composition is an increased ADCC activity and an increased binding activity to the epitope. In a further preferred embodiment the increased activity of an antibody molecule composition is an increased ADCC activity, an increased CDC activity, an increased binding activity to the epitope and an increased binding activity to at least one Fc receptor. In a further preferred embodiment the increased activity of an antibody molecule composition is an increased ADCC activity, increased cytotoxicity caused by phagocytosis, an increased binding activity to the epitope and an increased binding activity to at least one Fc receptor. In the most preferred embodiment the increased activity of an antibody molecule composition is an increased ADCC, increased cytotoxicity caused by phagocytosis, an increased CDC activity and an increased binding activity to the epitope and an increased binding activity to at least one Fc receptor.

In accordance with the present invention the term "improved homogeneity" means that an antibody molecule composition of the invention expressed in a host cell of human myeloid leukaemia origin of the invention comprises fewer different glycoforms, or more of a favourable glycoform or of favourable glycoforms, or less of at least one glycoform of an antibody molecule (preferably of those glycoforms which represent at least 1% of the total antibody molecule composition on its own) than at least one antibody molecule composition of the same antibody molecule isolated from at least one of the cell lines CHO, or CHOdhfr-, or BHK, or NS0, or SP2/0, or PerC.6 or mouse hybridoma when expressed therein. In a preferred embodiment of the invention it means that an antibody molecule composition of the invention expressed in a host cell of human myeloid leukaemia origin of the invention comprises fewer different glycoforms, or more of a favourable glycoform or of favourable glycoforms, or less of at least one glycoform of an antibody molecule than an antibody molecule composition of the same antibody molecule isolated from the cell line CHOdhfr- [ATCC No. CRL-9096] when expressed therein.

One heterogeneity particularly problematic for production and use in human is the sialylation. In a preferred embodiment the antibody molecule composition of the invention has an improved homogeneity by comprising no glycoform with the sialic acid N-glycolylneuraminic acid (NeuGc), whereby in this case no glycoform means no glycoform of more than 1% of all carbohydrate chains obtainable from the purified antibody molecule composition and more preferable no carbohydrate chain detectable at all as being detectable by the methods known to those skilled in the art. Since NeuGc is known to be able to be immunogenic in humans this is a large advantage of the host cells of the invention over other production systems such as CHO, NSO, SP2/0.

In a preferred embodiment the antibody molecule composition of the invention has an improved homogeneity in respect to sialylation by comprising less than 5% of glycoforms, more preferably less than 3%, even more preferably less than 1%, and most preferably no glycoforms of the antibody molecule composition with sialic acid detectable as described in examples. In a further preferred embodiment an antibody molecule composition with improved homogeneity in respect to sialylation is achieved by using a host cell of human myeloid leukaemia origin of the invention which has a defect in the sugar nucleotide precursor pathway and therefore is deficient for or has reduced CMP-sialic acid which results in no or a largely reduced sialylation of the carbohydrate sugar chains of the antibody molecules when the cells are grown in a serum-free medium. In an even further preferred embodiment such antibody molecule composition with improved homogeneity in respect to sialylation can be achieved by using NM-F9 [DSM ACC2606] or NM-D4 [DSM ACC2605] as a host cell of the invention grown in a serum-free medium as and described in more detail in examples.

In another further preferred embodiment an antibody molecule composition with improved homogeneity in respect to sialylation is achieved by using a host cell of human myeloid leukaemia origin of the invention which has a defect in the sugar nucleotide transporter of GMP-sialic acid or in at least one sialyltransferase which results in no or a reduced sialylation of the carbohydrate sugar chains of the antibody molecules when the cells are grown in a serum-free medium.

In another preferred embodiment of the invention the antibody molecule composition with improved homogeneity in respect to sialylation is achieved by using a host cell of human myeloid leukaemia origin of the invention which has an increased sialylation degree. Said sialylation degree means that the amount of the sialic acid N-acetylneuraminic (NeuNc or NeuNAc) on the antibody molecules in an antibody molecule composition is at least 5%, more preferably at least 15%, more preferably at least 20%, more preferably at least 25%, more preferably at least 30%, more preferably at least 35%, more preferably at least 40%, more preferably at least 50%, more preferably at least 60%, more preferably at least 70%, more preferably at least 80%, more preferably at least 90%, more preferably at least 100%, more preferably at least 3 times, more preferably at least 5 times, more preferably at least 10 times, more preferably at least 25 times, more preferably at least 50 times, and most preferably more than 100 times, higher than the amount of sialic acid N-acetylneuraminic (NeuNc or NeuNAc) of the total carbohydrate units or the particular carbohydrate chain at a particular Glycosyaltion site of the antibody molecule when comparing with the same amount of antibody molecules of an antibody molecule composition of the same antibody molecule isolated from at least one of the cell lines CHO, or CHOdhfr-, or BHK, or NS0, or SP2/0, or PerC.6 or mouse hybridoma, preferably CHOdhfr- [ATCC No. CRL-9096] when expressed therein. The sialylation degree can be detected by methods known to those skilled in the art, such as but not limited to immuno blot analysis or ELISA using lectins which binding depends on the sialylation of the carbohydrate structure, such as SNA, MAL, MAL I or PNA, by chemical detection methods such as the thiobarbituric acid method, by HPLC or mass spectrometry or combination thereof. Those skilled in the art can select the most suitable method and adopt and optimise it to the purpose and more details are described in the examples. Preferred is an immunoblot analysis using SNA. In another further preferred embodiment an antibody molecule composition with improved homogeneity in respect to sialylation is achieved by using a host cell of human myeloid leukaemia origin of the invention, preferably K562, NM-F9 [DSM ACC2606], NM-D4 [DSM ACC2605], NM-H9, H9, NM-H10, NM-E-2F9, NM-C-2F5, NM-H9D8, or NM-H9D8-E6,and cells or cell lines derived therefrom, and most preferably K562, NM-H9, H9, NM-H10, NM-E-2F9, NM-C-2F5, NM-H9D8, or NM-H9D8-E6, which has an increased sialylation degree, and results in a antibody molecule composition which comprises alpha 2-6 linked sialic acid, for detectable for example by SNA binding, in a more preferred version the antibody molecule composition comprises alpha 2-6 and alpha 2-3 linked sialic acids.

In another preferred embodiment an antibody molecule composition with improved homogeneity in respect to sialylation is achieved by using a host cell of human myeloid leukaemia origin which comprise at least one sialyltransferase able to attach NeuNAc in alpha 2-3 and at least one sialyltransferase able to attach NeuNAc in alpha 2-6 linkage to sugar groups, such as but not limited to K562, NM-F9 [DSM ACC2606], NM-D4 [DSM ACC2605], NM-H9, H9, NM-H10, NM-E-2F9, NM-C-2F5, NM-H9D8, or NM-H9D8-E6, and cells or cell lines derived therefrom, thereby resulting in a more preferred composition of glycoforms of the antibody molecule in the antibody molecule composition.

In an even further (preferred) embodiment of the invention said antibody molecule composition of the invention with improved homogeneity in respect to sialylation comprises at least one glycoform of an antibody molecule with at least one carbohydrate chain attached to another glycosylation site of the antibody molecule than the amino acid Asn-297 in the second domain (Cgamma2 domain) of the Fc region.

In an even further preferred embodiment of the former antibody molecule composition with improved homogeneity in respect to sialylation is expressed in a host cell of human myeloid leukaemia origin which has an increased sialylation degree and/or comprise at least one sialyltransferase able to attach sialic acid in alpha 2-3 and at least one sialyltransferase able to attach sialic acid in alpha 2-6 linkage to sugar groups, such as K562, NM-H9, H9, NM-H 10, NM-E-2F9, NM-C-2F5, NM-H9D8, or NM-H9D8-E6, and cells or cell lines derived therefrom.

In an even further preferred embodiment the former antibody molecule has at least one N-glycosylation site (Asn-X-Ser/Thr, whereby X can be any amino acid except Pro) and/or at least one O-glycosylation site in a sequence of the Fab region.

In a further preferred embodiment the antibody molecule composition of the invention with improved homogeneity in respect to sialylation comprising an antibody molecule which comprises at least one carbohydrate chain attached to another glycosylation site of the antibody molecule than the amino acid Asn-297 in the second domain (C$\gamma$2 domain) of the Fc part has an extended serum-half life and/or bioavailability when measured in at least one mammal such as mice, rats, or preferably in humans, than the antibody molecule composition of the same antibody molecule isolated from at least one of the cell lines CHO, or CHOdhfr-, or BHK, or NS0, or SP2/0, or PerC.6 or mouse hybridoma, preferably the cell line CHOdhfr- [ATCC No. CRL-9096] when expressed therein.

In an even more preferred embodiment the antibody molecule composition with improved homogeneity in respect to sialylation is expressed in a host cell of human myeloid leukaemia origin of the invention which has an increased sialylation degree and/or comprise at least one sialyltransferase able to attach sialic acid in alpha 2-3 and at least one sialyltransferase able to attach sialic acid in alpha 2-6 linkage to sugar groups, such as K562, NM-F9 [DSM ACC2606], NM-D4 [DSM ACC2605], NM-H9, H9, NM-H10, NM-E-2F9, NM-C-2F5, NM-H9D8, or NM-H9D8-E6, and cells or cell lines derived therefrom, and comprise at least one carbohydrate chain attached to at least one N-glycosylation site and/or at least one O-glycosylation site in a sequence of the Fab region of the antibody molecule and has an extended serum-half life and/or bioavailability when measured in at least one mammal such as mice, rats, or preferably in humans, than the antibody molecule composition of the same antibody molecule isolated from at least one of the cell lines CHO, or CHOdhfr-, or BHK, or NS0, or SP2/0, or PerC.6 or mouse hybridoma, preferably the cell line CHOdhfr- [ATCC No. CRL-9096] when expressed therein. In a further preferred embodiment the antibody molecule expressed is Erbitux (Cetuximab).

In accordance with the present invention the term "increased yield" means that the average or the maximum yield of an antibody molecule composition of the invention produced in a host cell of human myeloid leukaemia origin is higher than the respective average or maximum yield of at least one antibody molecule composition from the same antibody molecule when expressed in at least one of the cell lines CHO, or CHOdhfr-, or BHK, or NS0, or SP2/0, or PerC.6 or mouse hybridoma. In the preferred embodiment of the invention it means that the average or maximum yield of an antibody molecule composition of the invention expressed in a host cell of human myeloid leukaemia origin is higher than the respective average or maximum yield of an antibody molecule composition from the same antibody molecule when expressed in CHOdhfr- [ATCC No. CRL-9096] using the murine dhfr gene and methotrexate for amplification in CHOdhfr-. The average and maximum yield is measured in SPR, which reflects the productivity of a cell, cell mixture or a cell line, can be determined by those skilled in the art and is described in its preferred embodiment in the examples.

In a further preferred embodiment of the invention at least the average or the maximum yield of an antibody molecule composition of the invention expressed in a host cell of human myeloid leukaemia origin, preferably K562, NM-F9 [DSM ACC2606], NM-D4 [DSM ACC2605], NM-H9, H9, NM-H10, NM-E-2F9, NM-C-2F5, NM-H9D8, or NM-H9D8-E6, or a cell or cell line derived therefrom, is at least 10 % higher than the according of the antibody molecule composition from the same antibody molecule expressed in the cell line CHOdhfr- [ATCC No. CRL-9096] using the murine dhfr gene and methotrexate for amplification in CHOdhfr-, more preferably at least 15%, more preferably at least 20%, more preferably at least 25%, more preferably at least 30%, more preferably at least 35%, more preferably at least 45%, more preferably at least 50%, more preferably at least 55%, more preferably at least 60%, more preferably at least 70%, more preferably at least 80%, more preferably at least 90%, more preferably at least 100%, more preferably at least 3 times, more preferably at least 4 times, and most preferably more than 5 times.

The invention further provides a nucleic acid comprising

(a) a sequence encoding an antibody molecule or at least one part thereof as described elsewhere herein, and
(b) at least one sequence encoding a sequence from the group of sequence #1 to sequence #9

In a preferred embodiment of the invention the nucleic acid of the invention comprises

(a) a sequence encoding an antibody molecule or at least one part thereof as described elsewhere herein, and
(b) a sequence encoding sequence #1

In a further preferred embodiment of the invention the above described nucleic acid of the invention further comprises a sequence encoding a selection marker, preferably a sequence encoding for a polypeptide which induces an antibiotic resistance of a host cells in which said nucleic acid is introduced, such as but not limited to neomycin or puromycin.

In a further preferred embodiment of the invention the above described nucleic acid of the invention further comprises at least one sequence of at least one genetic element described elsewhere herein.

The invention further provides a host cell of human myeloid leukaemia origin or any human myeloid or myeloid precursor cell or cell line which can be obtained from a leukaemia patient, or any myeloid or myeloid precursor cell or cell line which can be obtained from a human donor or a mixture of cells or cell lines comprising at least one cell of human myeloid leukaemia origin, or cell, cells, or cell line which was obtained by fusing at least one cell, cells, or a cell line of human myeloid leukaemia origin or any human myeloid or myeloid precursor cell or cell line which can be obtained from a leukaemia patient, or any myeloid or myeloid precursor cell or cell line which can be obtained from a human donor, with another cell of human or animal origin, such as but not limited to B cells, CHO cells, comprising at least one nucleic acid encoding an antibody molecule or parts thereof which was introduced into said cells.

The invention provides a host cell of the invention described above comprising at least one nucleic acid encoding an antibody molecule or at least one part thereof.

In a preferred embodiment the invention provides the host cell K562, NM-F9 [DSM ACC2606], NM-D4 [DSM ACC2605], NM-H9, H9, NM-H10, NM-E-2F9, NM-C-2F5, NM-H9D8, or NM-H9D8-E6, or a cell or cell line derived from anyone of said host cells, preferably those which grows under serum-free conditions and from which an antibody molecule composition is isolated under serum-free conditions, and even more preferred those into which the nucleic acid encoding the antibody molecule was introduced under serum-free conditions, comprising at least one nucleic acid encoding an antibody molecule or parts thereof, preferably a nucleic acid comprising a sequence encoding an antibody molecule or at least one part thereof as described elsewhere herein.

The invention provides a host cell of the invention described above comprising at least one nucleic acid encoding at least one polypeptide of the group of sequence #1 to sequence #9, preferably sequence #1.

The invention provides a host cell of the invention described above comprising at least one nucleic acid encoding an antibody molecule or parts thereof and at least one nucleic acid encoding at least one polypeptide of the group of sequence #1 to sequence #9, preferably sequence #1.

In a preferred embodiment the invention provides the host cell K562, NM-F9 [DSM ACC2606], NM-D4 [DSM ACC2605], NM-H9, H9, NM-H10, NM-E-2F9, NM-C-2F5, NM-H9D8, or NM-H9D8-E6, or a cell or cell line derived from anyone of said host cells, preferably those which grows under serum-free conditions and from which an antibody molecule composition is isolated under serum-free conditions, and even more preferred those into which the nucleic acid encoding the antibody molecule was introduced under serum-free conditions, comprising at least one nucleic acid encoding an antibody molecule or parts thereof, preferably a nucleic acid comprising a sequence encoding an antibody molecule or at least one part thereof as described elsewhere herein, and at least one sequence encoding a sequence from the group of sequence #1 to sequence #9, preferably sequence #1.

In a further preferred embodiment the invention provides the above described host cells wherein the antibody molecule encoded is an antibody of WO2004/065423.

In an even further preferred embodiment the invention provides the above described host cells wherein the antibody molecule encoded is the antibody PankoMab [Cancer Immunol Immunother. 2006 Nov;55(11):1337-47. Epub 2006 Feb. PankoMab: a potent new generation anti-tumour MUC1 antibody. Danielczyk et al], preferably a chimaeric form of PankoMab with all human constant domains, and more preferably a humanized PankoMab.

The invention further provides an antibody molecule composition having increased activity and/or increased yield and/or improved homogeneity and fully human glycosylation produced by any of the methods of the invention as described somewhere herein.

In a preferred embodiment the invention provides an antibody molecule composition produced by any of the methods of the invention has an increased activity and/or increased yield and/or improved homogeneity and fully human glycosylation produced by any of the methods of the invention when compared to an antibody molecule composition of the same antibody molecule isolated from at least one of the cell lines CHO, or CHOdhfr-, or BHK, or NS0, or SP2/0, or PerC.6 or mouse hybridoma, preferably CHOdhfr- [ATCC No. CRL-9096], when expressed therein.

The antibody molecule or part thereof expressed can be any antibody or antibody part or antibody fragment.

In a preferred embodiment of the invention the antibody molecule expressed is an antibody molecule recognizing psoriasis, rheumatoid arthritis, Crohn's disease, ulcerative colitis, autoimmune diseases, SLE, Multiple Sclerosis, autoimmune haematological disorders, asthma, allergy, graft-versus-host disease, allograft rejection, glaucoma surgery, myocardial infarction, viruses as RSV, HIV, Hep B, or CMV, cancer, sarcoma, CLL, AML, or NHL.

In an even preferred embodiment of the invention the antibody molecule expressed is an antibody molecule recognizing the cancer, tumor or metastasis, at least one cancer cell or tumor cell, in at least one human, preferably selected from the group of cancerous diseases or tumor diseases of the ear-nose-throat region, of the lungs, mediastinum, gastrointestinal tract, urogenital system, gynecological system, breast, endocrine system, skin, bone and soft-tissue sarcomas, mesotheliomas, melanomas, neoplasms of the central nervous system, cancerous diseases or tumor diseases during infancy, lymphomas, leukemias, paraneoplastic syndromes, metastases with unknown primary tumor (CUP syndrome), peritoneal carcinomatoses, immunosuppression-related malignancies and/or tumor metastases.

In a preferred embodiment of the invention the expressed antibodies or parts thereof are an anti MUC1 antibody.

In a further preferred embodiment of the invention the antibody molecule encoded by the nucleic acid or nucleic acids of the invention is the antibody Rituximab, Herceptin, Erbitux, Campath 1H or antibodies derived therefrom.

In an even more preferred embodiment of the invention the antibody molecule encoded by the nucleic acid or nucleic acids of the invention is an antibody of WO2004/050707, and even more preferred of WO2004/065423, and even more preferred PankoMab [Cancer Immunol Immunother. 2006 Nov;55(11):1337-47. Epub 2006 Feb. PankoMab: a potent new generation anti-tumour MUC1 antibody. Danielczyk et al], and even more preferred a chimaeric version thereof comprising all human constant domains, and even more preferred a humanized antibody thereof.

In an even more preferred embodiment of the invention the antibody molecule encoded by the nucleic acid or nucleic acids of the invention is any whole antibody of the invention, preferably Rituximab, Herceptin, Erbitux, more preferably WO2004/065423, most preferably PankoMab, whereby the antibody molecule composition isolated from any host cell of the invention, preferably K562, NM-F9 [DSM ACC2606], NM-D4 [DSM ACC2605], NM-H9, H9, NM-H10, NM-E-2F9, NM-C-2F5, NM-H9D8, or NM-H9D8-E6, or a cell or cell line derived from anyone of said host cells, comprises no detectable NeuGc.

In an even more preferred embodiment of the invention the antibody molecule encoded by the nucleic acid or nucleic acids of the invention is any whole antibody molecule or an antibody molecule comprising the Cgamma2 domain of the invention, preferably Rituximab, Herceptin, Erbitux, more preferably WO2004/065423, most preferably PankoMab, whereby the antibody molecule composition isolated from a host cell of the invention, preferably K562, NM-F9 [DSM ACC2606], NM-D4 [DSM ACC2605], NM-H9, H9, NM-H10, NM-E-2F9, NM-C-2F5, NM-H9D8, or NM-H9D8-E6, or a cell or cell line derived from anyone of said host cells, comprises at least one glycoform with alpha 2-6 sialic acid.

In an even more preferred embodiment of the invention the antibody molecule encoded by the nucleic acid or nucleic acids of the invention is any whole antibody molecule or an antibody molecule comprising the Cgamma2 domain of the invention, preferably Rituximab, Herceptin, Erbitux, Campath 1H, more preferably WO2004/065423, most preferably PankoMab, whereby the antibody molecule composition isolated from host cell NM-H9, H9, NM-H10, NM-E-2F9, NM-C-2F5, NM-H9D8, or NM-H9D8-E6, or a cell or cell line derived from anyone of said host cells, comprises alpha 2-6 sialic acid detectable by immune blot analysis with the lectin SNA as described in examples.

In another preferred embodiment of the invention the antibody molecule encoded by the nucleic acid or nucleic acids of the invention is any antibody molecule, preferably Rituximab, Herceptin, Erbitux, Campath 1H, more preferably WO2004/065423, most preferably PankoMab, and the antibody molecule composition isolated from the host cell NM-F9 or NM-D4 of the invention after cultivation in serum-free and more preferably protein-free medium has an improved homogeneity with no detectable sialic acids.

In a further preferred embodiment of the invention the antibody molecule encoded by the nucleic acid or nucleic acids of the invention is the antibody Rituximab, Herceptin, Campath 1H, or antibodies derived therefrom, and the antibody molecule composition isolated from the host cell of the invention has an increased ADCC activity of at least 4 fold higher than the activity of the antibody molecule composition from the same antibody molecule expressed in the cell line CHOdhfr- [ATCC No. CRL-9096].

In an even more preferred embodiment of the invention the antibody molecule encoded by the nucleic acid or nucleic acids of the invention is an antibody of WO2004/065423, more preferably PankoMab, and the antibody molecule composition isolated from the host cell of the invention has an increased ADCC activity of at least 4 fold higher when produced in at least one of the cells K562, NM-F9 [DSM ACC2606], NM-D4 [DSM ACC2605], NM-H9, H9, NM-H10, NM-E-2F9, NM-C-2F5, NM-H9D8, or NM-H9D8-E6, or a cell or cell line derived from anyone of said host cells than the activity of the antibody molecule composition from the same antibody molecule expressed in the cell line CHOdhfr- [ATCC No. CRL-9096].

In another preferred embodiment of the invention the antibody molecule encoded by the nucleic acid or nucleic acids of the invention is an antibody of WO2004/065423, more preferably PankoMab, and the antibody molecule composition isolated from the host cell of the invention K562, NM-F9 [DSM ACC2606], NM-D4 [DSM ACC2605], NM-H9, H9, NM-H10, NM-E-2F9, NM-C-2F5, NM-H9D8, or NM-H9D8-E6, or a cell or cell line derived from anyone of said host cells has an increased binding activity to its epitope of at least 50% higher, preferably 2 fold higher than the activity of the antibody molecule composition from the same antibody molecule expressed in the cell line CHOdhfr- [ATCC No. CRL-9096].

In another preferred embodiment of the invention the antibody molecule encoded by the nucleic acid or nucleic acids of the invention is an antibody of WO2004/065423, more preferably PankoMab, and the antibody molecule composition isolated from the host cell NM-F9 or NM-D4 of the invention after cultivation in serum-free and more preferably protein-free medium has an improved homogeneity with no detectable sialic acids.

In another preferred embodiment of the invention the antibody molecule encoded by the nucleic acid or nucleic acids of the invention is an antibody of WO2004/065423, more preferably PankoMab, and the antibody molecule composition isolated from the host cell K562, NM-F9 [DSM ACC2606], NM-D4 [DSM ACC2605], NM-H9, H9, NM-H10, NM-E-2F9, NM-C-2F5, NM-H9D8, or NM-H9D8-E6, or a cell or cell line derived from anyone of said host cells has an improved homogeneity with at least 10% more sialic acids than the antibody molecule composition from the same antibody molecule expressed in the cell line CHOdhfr- [ATCC No. CRL-9096].

In another preferred embodiment of the invention the antibody molecule encoded by the nucleic acid or nucleic acids of the invention is an antibody of WO2004/065423, more preferably PankoMab, and the antibody molecule composition isolated from the host cell of the invention has an improved homogeneity with a higher degree of no detectable sialic acids when expressed in NM-F9 or NM-D4 when compared to an antibody molecule composition from the same antibody molecule expressed in the cell line CHOdhfr- [ATCC No. CRL-9096].

In another preferred embodiment of the invention the antibody molecule encoded by the nucleic acid or nucleic acids of the invention is an antibody of WO2004/065423, more preferably PankoMab, and the antibody molecule composition isolated from the host cell of the invention has an improved homogeneity as described above, and an increased ADCC activity of at least 4 fold higher than the activity of the antibody molecule composition from the same antibody molecule expressed in the cell line CHOdhfr- [ATCC No. CRL-9096].

In the most preferred embodiment of the invention the antibody molecule encoded by the nucleic acid or nucleic acids of the invention is an antibody of WO2004/065423, more preferably PankoMab.

The invention further provides a host cell for producing an antibody molecule composition having increased activity and/or increased yield and/or improved homogeneity and fully human glycosylation in sense of the invention as described elsewhere herein, wherein the host cell is any cell, cells, or cell line of human myeloid leukaemia origin or any human myeloid or myeloid precursor cell or cell line which can be obtained from a leukaemia patient, or any myeloid or myeloid precursor cell or cell line which can be obtained from a human donor or a mixture of cells or cell lines comprising at least one cell of human myeloid leukaemia origin, or a cell or cell line derived therefrom as described elsewhere herein, or a mixture of cells or cell lines comprising at least one of those aforementioned cells.

The invention also provides a host cell for producing an antibody molecule composition having increased activity and/or increased yield and/or improved homogeneity and fully human glycosylation in sense of the invention as described elsewhere herein, wherein the host cell is any cell, cells, or cell line which was obtained by fusing at least one cell, cells, or a cell line of human myeloid leukaemia origin or any human myeloid or myeloid precursor cell or cell line which can be obtained from a leukaemia patient, or any myeloid or myeloid precursor cell or cell line which can be obtained from a human donor, with another cell of human or animal origin, such as but not limited to B cells, CHO cells.

In a preferred embodiment said host cell the invention provides for producing an antibody molecule composition having increased activity and/or increased yield and/or improved homogeneity in sense of the invention as described elsewhere herein, is the cell or cell line KG1, MUTZ-3, K562, NM-F9 [DSM ACC2606], NM-D4 [DSM ACC2605], NM-H9, H9, NM-H10, NM-E-2F9, NM-C-2F5, NM-H9D8, or NM-H9D8-E6, or a cell or cell line derived therefrom, or a mixture of cells or cell lines comprising at least one of those aforementioned cells.

In a further preferred embodiment said host cell the invention provides for producing an antibody molecule composition having increased activity and/or increased yield and/or improved homogeneity in sense of the invention as described elsewhere herein, is the cell or cell line K562, NM-F9 [DSM ACC2606], NM-D4 [DSM ACC2605], NM-H9, H9, NM-H10, NM-E-2F9, NM-C-2F5, NM-H9D8, or NM-H9D8-E6, or a cell or cell line derived therefrom.

In a further preferred embodiment said host cell the invention provides for producing an antibody molecule composition having increased activity and/or increased yield and/or improved homogeneity in sense of the invention as described elsewhere herein, is the cell or cell line NM-F9 [DSM ACC2606], NM-D4 [DSM ACC2605], NM-H9, H9, NM-H10, NM-E-2F9, NM-C-2F5, NM-H9D8, or NM-H9D8-E6, or a cell or cell line derived therefrom or a cell or cell line derived therefrom as described elsewhere herein.

In an even further preferred embodiment said host cell the invention provides for producing an antibody molecule composition having increased activity and/or increased yield and/or improved homogeneity in sense of the invention as described elsewhere herein, is a cell or a cell line derived from KG1, MUTZ-3, K562, NM-F9 [DSM ACC2606], NM-D4 [DSM ACC2605], NM-H9, H9, NM-H10, NM-E-2F9, NM-C-2F5, NM-H9D8, or NM-H9D8-E6, or a cell or cell line derived therefrom as described elsewhere herein, which grows under serum-free conditions, and preferably those in which the nucleic acid encoding the antibody molecule can be introduced in these cells and an antibody molecule composition is isolated under serum-free conditions.

In an even further preferred embodiment said host cell the invention provides for producing an antibody molecule composition having increased activity and/or increased yield and/or improved homogeneity in sense of the invention as described elsewhere herein, is a cell or a cell line derived from K562, NM-F9 [DSM ACC2606], NM-D4 [DSM ACC2605], NM-H9, H9, NM-H10, NM-E-2F9, NM-C-2F5, NM-H9D8, or NM-H9D8-E6, or a cell or cell line derived therefrom as described elsewhere herein, which grows under serum-free conditions.

In an even further preferred embodiment said host cell the invention provides for producing an antibody molecule composition having increased activity and/or increased yield and/or improved homogeneity in sense of the invention as described elsewhere herein, is a cell or a cell line derived from K562, NM-F9 [DSM ACC2606], NM-D4 [DSM ACC2605], NM-H9, H9, NM-H10, NM-E-2F9, NM-C-2F5, NM-H9D8, or NM-H9D8-E6, or a cell or cell line derived therefrom as described elsewhere herein, which grows under serum-free conditions and in which the nucleic acid encoding the antibody molecule can be introduced in these cells and an antibody molecule composition is isolated under serum-free conditions.

In the most preferred embodiment said host cell the invention provides for producing an antibody molecule composition having increased activity and/or increased yield and/or improved homogeneity in sense of the invention as described elsewhere herein, is the cell or cell line NM-F9 [DSM ACC2606], NM-D4 [DSM ACC2605], NM-H9, H9, NM-H10, NM-E-2F9, NM-C-2F5, NM-H9D8, or NM-H9D8-E6, or a cell or cell line derived therefrom as described elsewhere herein, which grows under serum-free conditions, and preferably those in which the nucleic acid encoding the antibody molecule can be introduced in these cells and an antibody molecule composition is isolated under serum-free conditions.

The invention further provides an antibody molecule composition isolated by any of the methods of the invention described elsewhere herein.

The invention further provides an antibody molecule composition isolated by any of the methods of the invention described elsewhere herein which has an increased activity and/or increased yield and/or improved homogeneity in sense of the invention and described elsewhere herein.

Examples of such antibodies include antibodies against ganglioside GD3, human interleukin-5 receptor alpha -chain, HER2, CC chemokine receptor 4, CD20, CD22, neuroblastoma, MUC 1, epidermal growth factor receptor (EGFR).

In a preferred embodiment of the invention said antibody molecule composition originates from any of the antibody molecule of the group of Muromomab, Daclizumab, Basiliximab, Abciximab, Rituximab, Herceptin, Gemtuzumab, Alemtuzumab, Ibritumomab, Cetuximab (Erbitux), Bevacizumab, Tositumomab, Pavlizumab, Infliximab, Eculizumab, Epratuzumab, Omalizumab, Efalizumab, Adalimumab, Campath-1H, C2B8, Panorex, BrevaRex, Simulect, Antova, OKT3, Zenapax, ReoPro, Synagis, Ostavir, Protovir, OvaRex, Vitaxin.

In a more preferred embodiment of the invention said antibody molecule composition originates from any of the antibody molecule of the group of Rituximab, Herceptin, anti-CC chemokine receptor 4 antibody KM2160, Campath-1H, C2B8, Erbitux, anti-neuroblastoma antibody chCE7.In an even more preferred embodiment of the invention said antibody molecule composition originates from the antibody molecule of the group of WO2004/065423, more preferably PankoMab, more preferably its chimaeric and even more preferably its humanized form.

The following tables 1 and 2 and figures 1 to 4 illustrate the present invention.

Table 1: Yield of chimaeric PankoMab expressed in CHOdhfr- and NM-F9 cultured in medium supplemented with FCS.

Table 2: Yield of chimaeric PankoMab expressed in NM-H9D8 cultured in serum-free medium.

Figure 1: fut8 mRNA expression of NM-F9, NM-D4, and NM-H9D8 cell. As a control served HepG2 cells.

Figure 2: ADCC activity of chimaeric PankoMab isolated from NM-F9 is ~5 times higher than chimaeric PankoMab isolated from the CHOdhfr- cells.

Figure 3: Binding activity of the chimaeric PankoMab isolated from NM-F9 to synthetic glycosylated MUC1 30-mer peptide is about 50% higher than the binding activity of the chimaeric PankoMab isolated from the CHOdhfr-cells.

Figure 4:  Western blot analysis was performed to identify the differently sialylated heavy chain of antibody molecule compositions expressed in CHOdhfr-, NM-F9, or NM-H9D8. Proteins were transfered to nitrocellulose and visualized either by secondary anti-human IgG antibodies (Fig. 4A) or SNA (Fig. 4B) which detects 2-6 sialylation.

**Example 1**

**Analyses of fut8 expression in cells**

RNA was extracted from NM-F9 (DSM ACC2606), NM-D4 (DSM ACC2605), NM-H9, K562, NM-H9D8 and HepG2 (control) cells according to standard procedures (RNeasy-Mini-Kit, Qiagen). The mRNA was isolated using magnetic beads technology according to manufacturers instruction (Dynabeads® Oligo(dT)25, Invitrogen) For the first-strand cDNA synthesis, 50 $\mu$l bead suspension of each sample and Omniscript Reverse Transcriptase (Qiagen) were used according to manufacturers instructions. For the subsequent RT-PCR reaction 5 $\mu$l of the cDNA product and specific *fut8* primers were used resulting in a 212bp fragment. As a control actin specific primers were used resulting in 240bp fragment. The resulting PCR-product(s) were analysed on a 1.5% gel.
NM-F9, NM-D4, NM-H9, K562, NM-H9D8 do express the mRNA for *fut8.*

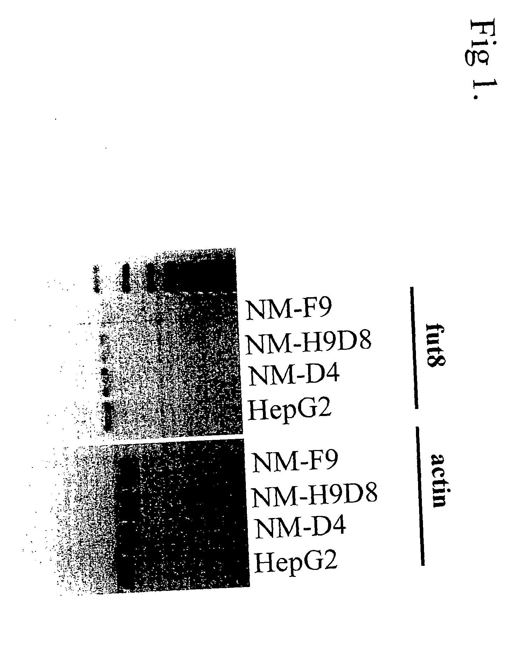

Figure 1 shows as an example the fut8 mRNA expression of NM-F9, NM-D4, and NM-H9D8.

**Example 2**

**Glycoengineering of K562 cells**

Glycoengineering of K562 cells and generation of NM-D4 and NM-F9 cell line are described in EP1654353.
NM-H9 cells, and a cell or cell line derived therefrom with high sialylation potential were generated as follows. Random mutagenesis was performed by treating K562 cells with the alkylating agent ethyl methanesulfonate. Per sample K562 cells were washed in PBS and seeded at $10^6$ cells per ml cell culture medium supplemented with EMS (0.1 mg/ml, ethyl methanesulfonate, Sigma-Aldrich) overnight at 37 °C and 5 % $CO_2$. Cells were washed and provided with fresh medium. Every second day cell vitality was determined by trypan blue staining, and cells were analyzed by immunocytochemical staining.
Subsequently, cells exposing the novel phenotype of high TF expression were selected by means of a TF-specific antibody. K562 cells were washed in B-PBS (0.5 % BSA in PBS), incubated with 50 $\mu$l of supernatant of hybridoma cultures of the monoclonal antibody A78-G/A7 or PankoMab and 950 $\mu$l of B-PBS at 4°C for 30 min. After washing the procedure was repeated with 50 $\mu$l of rat-anti-mouse-IgM-antibody or rat-anti-mouse-IgG-antibody conjugated with MicroBeads (Miltenyi Biotec, Köln, Germany). After washing the magnetically labeled TF-positive K562 cells were separated by two successive columns provided by Miltenyi Biotec (Köln, Germany) as described in the manufacturers manual. Following nine days of cultivation, the isolation procedure was repeated in total three times. FACS analysis (flow cytometry) started with antibody staining: About 3x$10^5$ cells were incubated at 4°C for 1.5 h with primary monoclonal antibody (hybridoma culture supernatants of A78-G/A7 (IgM), PankoMab (IgG1), all diluted 1:2 in cell culture medium) followed by the secondary Cy3-conjugated goat anti-mouse IgM or IgG antibody 1:200 diluted in PBS, at 4°C for 30 min and were washed again. Resuspended cells (200 $\mu$l PBS) were investigated by flow cytometry (flow cytometer: Coulter Epics, Beckman Coulter, Krefeld, Ger). Quantitative analyses were carried out using the Expo32 software (Becton Coulter) with following parameter for antibody labeled cells: forward scatter (FS): 26 V, gain 1, sideward scatter (SS): 807 V, gain 5, FL2: 740 V, gain 1, and following parameter for lectin labeled cells: FS: 26 V, gain 1, SS: 807 V, gain 5, FL1:740 V, gain 1).
After three rounds of isolation a K562 cell population of 93 % TF-positive cells was received. However, the percentage of TF-positive K562 cells decreased over time reaching a bottom level of about 20 % TF-positive cells during a period of 14 days following the isolation procedure. For stable expression of the TF-positive phenotype, K562 cells were isolated for a forth time and finally, the isolated TF-positive K562 cells were cloned thereafter by limited dilution in 96-well plates (1 cell /100 $\mu$l). Among thirty K562 cell clones that were obtained, seventeen cell clones expressed low amounts of the TF antigen or no TF antigen. These cell clones were analysed for SNA binding in flow cytometry and for prolifaration rates (analysis of doubling time see below). Cell clones showing high SNA binding and high proliferation rate were selected. Stable NM-H9 clone was selected for further clone development by single cell cloning and to optimise growth under serum-free conditions. As the most preferred cell clone NM-H9D8 was selected and deposited under DSM ACC_____ at the "DSMZ-Deutsche Sammlung von Mikroorganismen und Zellkulturen GmbH" in Braunschweig (Germany), by Glycotope GmbH, Robert-Rössle-Str. 10, 13125 Berlin (Germany) at the September 15, 2006.

**Example 3**

**Cultivation of K562, NM-F9, NM-D4, NM-H9 and CHOdhfr- cells and generation of serum free cell lines**

K562 (ATCC CCL-243), NM-F9 (DSM ACC2606), NM-H9, NM-H9D8, and NM-D4 (DSM ACC2605) were cultured in RPMI 1640 supplemented with 10% FCS and 2 mM glutamine or serum free in X-Vivo 20 medium and grown at 37°C in a humidified atmosphere of 8%. CHOdhfr- cells (ATCC No. CRL-9096) were cultured in DMEM supplemented with 10% FCS, 2 mM glutamine, and 2% HT supplement or serum free in CHO-S-SFM II medium and grown at 37°C in a humidified atmosphere of 8%.

K562, NM-F9, NM-D4, NM-H9, NM-H9, H9, NM-H10, NM-E-2F9, NM-C-2F5, NM-H9D8, or NM-H9D8-E6, and cell or cell line derived from said host cells are easily adapted to serum free conditions by a complete change of cell culture medium. Cells and cell lines according to the present invention are inoculated into a serum-free medium as X-Vivo 20 at a density of $1 \times 10^5$ to $5 \times 10^5$ cells/ml, preferably $2 \times 10^5$ cells/ml, and cultured by an ordinary culturing method for animal cells. After 4 to 7 days of culturing, the cells whose density reached $5 \times 10^5$ to $10 \times 10^5$ cells/ml are selected as the cells adapted to a serum-free medium. Adaptation to serum-free media can be also performed by successive dilution of the medium supplemented with FCS by a serum-free medium composition (continuous adaptation). Productivity of converted FCS production clones of antibody composition producing host cells of the present invention to serum free conditions is mostly preserved.

Adaptation of CHOdhfr- (ATCC No. CRL-9096) to serum free conditions has to be performed stepwise and takes several weeks whereby a lost of productivity of at least a half is usual.

**Example 4**

**Cloning of vectors to express chimeric PankoMab in eukaryotic cells**

Variable sequences of PankoMab were PCR amplified with specific primers from the murine hybridoma cells producing PankoMab [Cancer Immunol Immunother. 2006 Nov;55(11):1337-47. Epub 2006 Feb. PankoMab: a potent new generation anti-tumour MUC1 antibody. Danielczyk et al]. The NcoI/XhoI-cut variable heavy chain fragment VH was cloned into the NcoI/SalI-cut BS-Leader vector as described in WO2004/065423. The BS-Leader vector includes a cloning cassette to introduce the T cell receptor signal peptide sequence at the 5' end and a splice donor sequence at the 3' end of the variable domains. The variable light chain VL of the corresponding antibody was amplified with a specific primer at the 3' end encoding additionally the splice donor site and was cloned via NcoI/NheI into the likewise digested BS-Leader vector. Thereafter, each HindIII/BamHI fragment from the BS-Leader vector was cloned into the corresponding eukaryotic expression vector. These vectors (pEFpuroC$\gamma$1V$_H$, pEFdhfrC$\kappa$V$_L$, pEFdhfr$_{mut}$C$\kappa$V$_L$) comprise EF-1$\alpha$-promoter and HCMV enhancer, SV40 origin, polyadenylation signal, puromycin resistance gene in the vector for the heavy chain and the murine dihydrofolase gene (dhfr) for CHO cell expression or SEQ ID 1 (Sequence 1#) for NM-F9, K562, NM-H9, NM-D4, or NM-H9D8 expression for selection and gene amplification in the vector for the light chain, as well as the genomic sequences of the human constant $\gamma$1 region for the heavy chain or the human constant $\kappa$ region for the light chain (primers for amplification from genomic human DNA and vector map see WO2004/065423).

**Example 5**

**Transfection of eukaryotic cells to express chimeric PankoMab and gene amplification procedure to generate high producing cell clones in the presence of serum**

To express the chimeric PankoMab in NM-F9 (DSM ACC2606), and CHOdhfr- (ATCC No. CRL-9096) cells were co-transfected with a mixture of above described vectors for the heavy and light chains (1:1 to 1:3) by lipofection using DMRIE-C or electroporation for the suspension cells as NM-F9 and lipofectamin or electroporation for the adherent cell line CHOdhfr-. Two days post-transfection, growth medium was changed to selection medium (NM-F9 in RPMI 1640 + 10% FCS + 2 mM L-glutamine + 0.75 $\mu$g/ml puromycin + 50 nM methotrexate; CHOdhfr- in DMEM + 10% dialysed FCS + 2 mM L-glutamine + 5 $\mu$g/ml puromycin + 50 mM methotrexate) for 1 week. First amplification was performed by increasing methotrexate concentration to 100 nM for additional 2 weeks. Part of amplified cell population was single cell cloned in medium without addition of puromycin and methotrexate, rest of the cells were subjected to a new round of gene amplification by increasing the methotrexate concentration. In this manner four to six rounds of gene amplification (100, 200, 500, 1000, 2000, 3000 nM methotrexate) were performed. Additionally, best producing clones identified by clone screening and analysis were amplified further similarly.

Following single cell cloning in 96-well plates using limited dilution (0.5 cells per well), plates were cultivated for 2 to 3 weeks, microscopically analysed for growing cell clones, and cloning efficiency in % (number of wells with growing cell clones x 100 / theoretical number of seeded cells) was determined. Growing clones were screened for productivity using different procedures for adherent CHOdhfr- cells or for NM-F9 suspension cells.

**CHOdhfr-:** Cells of growing clones were washed with PBS and harvested by Accutase treatment. Half of resuspended cells were seeded in a 96-well test plate, the other half was seeded in a 24-well plate for further cultivation. Test plate was cultivated for 20 to 24 h. Supernatant of each well was analysed for antibody titre as described in Determination of specific productivity rate (SPR) and doubling time (g) (see below). Relative cell density was measured using the MTT assay. In detail, cell were incubated with MTT solution for 2 h, solution was discarded and cell lysed by a 0.04 M HCl solution in 2-propanol. After 2 hours plate was moderately mixed and measured using a microtiter plate photometer with 570 nm filter in dual mode versus 630 nm reference filter.

**NM-F9:** 96-well plates were centrifuged and supernatant was discarded. Cell were resuspended in 200 $\mu$l fresh medium. Half of resuspended cells were seeded in a 96-well test plate and diluted with 100 $\mu$l medium, the other half remains in the cloning plates for further cultivation. After 2 days of cultivation, test plate was centrifuged and 20 $\mu$l of supernatant were analysed for antibody titre as described in Determination of specific productivity rate (SPR) and doubling time (g) (see below).

Relative cell density was measured using the WST-1 assay by addition of 10 $\mu$l WST-1 solution (Roche) in each well. After 1 to 3 hours incubation measurement was performed using a microtiter plate photometer with 450 nm filter in dual mode versus 630 nm reference filter.

Cell clones with a high ratio of antibody titre to cell density were selected and cultivated and analysed further.

The conditions for K562, NM-D4 and NM-H9 were the same.

Determination of specific productivity rate (SPR) and doubling time (g)

For each clone, $2 \times 10^4$ cells were seeded per well of a 24-well tissue culture plate in 500 $\mu$l growth media. The cells were allowed to grow for 3 days, conditioned media harvested for analysis, and the cells were removed if necessary by Accutase and counted. Specific antibody titres were quantitatively determined from media samples by ELISA. Assay plates were coated with an human kappa chain specific antibody (BD). Bound recombinant antibody was detected with anti-human IgG (H+L) horseradish peroxidase (HRP) conjugate (Jackson Immunoresearch Laboratories). For the quantification, purified recombinant chimeric antibody was used as a standard.

The SPR measured in picograms of specific protein per cell per day (pcd) is a function of both growth rate and productivity, and was calculated by following equations:

$$SPR = \frac{total\ protein\ mass}{integral\ cell\ area\ (ICA)}$$

$$ICA = \frac{(final\ cell\ number - initial\ cell\ number)\ x\ days\ in\ culture}{log_e\ (final\ cell\ number\ /\ initial\ cell\ number)}$$

Doubling time was calculated by following equation:

$$g = log\ 2x(hours\ in\ culture)\ /\ log\ (final\ cell\ number\ /\ initial\ cell\ number)$$

Determination of average yield and maximum yield for the cell lines

Following single cell cloning in 96-well plates using limited dilution (0.5 cells per well) as described above, from 200 theoretically plated single clones, the SPR is determined for growing cell clones and the average and deviation is determined, as well as the maximum yield for the different cell lines and different conditions. Table 1 compares the data of chimeric PankoMab-producing cell clones of CHOdhfr- and NM-F9 developed under serum-containing conditions.

**Example 6**

**Generation of serum-free high yield cell clones producing antibody molecule composition**

Transfection of NM-H9D8 adapted to serum-free medium X-Vivo 20 was performed under serum-free conditions using electroporation. Two days post-transfection, growth medium was changed to selection medium (X-Vivo 20 + 0.75 μg/ml puromycin + 50 nM methotrexate) for 1 week. First amplification was performed by increasing methotrexate concentration to 100 nM for additional 2 weeks. Part of amplified cell population was single cell cloned in X-Vivo without addition of puromycin and methotrexate, rest of the cells were subjected to a new round of gene amplification by increasing the methotrexate concentration. In this manner four to six rounds of gene amplification (100, 200, 500, 1000, 2000, 3000 nM methotrexate) were performed. Additionally, best producing clones identified by clone screening and analysis were amplified further similarly. Following single cell cloning in 96-well plates using limited dilution (0.5 cells per well), plates were cultivated for 2 to 3 weeks, microscopically analysed for growing cell clones, and cloning efficiency in % (number of wells with growing cell clones x 100 / theoretical number of seeded cells) was determined. Growing clones were screened for productivity using the same procedure as for NM-F9 suspension cells in the presence of serum (see above).

Determination of average yield and maximum yield for the cell clones

Following single cell cloning in 96-well plates using limited dilution (0.5 cells per well) as described above, from 200 theoretically plated single clones, the SPR is determined for growing cell clones and the average and deviation is determined, as well as the maximum yield for different conditions. Table 2 shows data of chimeric PankoMab-producing cell clones of NM-H9D8 developed completely under serum-free conditions.

Specific production rates (SPR) following amplification in cell lines according to the invention are higher than in CHOdhfr-, and highest in chimeric PankoMab-producing NM-H9D8 cells developed under serum-free conditions. The production rate of the majority of clones is highly stable over at least 6 weeks without selection pressure. Best clone was stable with a SPR of 30 pcd over 6 weeks with a doubling rate of 24 h.

Date reflects productivity of clones in small-lab scale with further potential in productivity increase by process development, media optimisation and fermentation.

Higher productivity and yield of production clones developed under serum-free conditions are advantageous and conditions are the same for all other antibodies and for all cell lines as K562, NM-F9 [DSM ACC2606], NM-D4 [DSM ACC2605], NM-H9, H9, NM-H10, NM-E-2F9, NM-C-2F5, or NM-H9D8-E6 adapted to serum-free conditions.

**Example 7**

**Isolation of an antibody molecule composition**

For production and isolation of an antibody molecule composition according to the invention, the stably transfected cells secreting the chimeric PankoMab were cultivated in serum free medium until a cell density of about 1 to 2 x $10^6$ cells/ml was reached. Following removal of the cells from the cell culture supernatant by centrifugation (400 x g, 15 min), the chimeric antibody was purified using a protein A column (HiTrap r-protein A FF, Amersham Pharmacia Biotech). The purified antibody fraction eluted by sudden pH change was re-buffered in PBS and concentrated using Centriprep centrifuge tubes (cut off 50 kDa, Millipore).

**Example 8**

**Determination of <u>Fc-mediated cellular cytotoxicity</u> of antibody molecule compositions accordimg to the invention**

**PBMC isolation from blood donors as effector cells**

PBMC were isolated from blood of healthy donors by density centrifugation with Ficoll-Hypaque (Biochrom). Cells were washed 3 times with RPMI 1640 supplemented with 5% FCS and cryopreserved in separate batches of $5x10^7$ cells. PBMC were thawed and used directly or kept overnight in RPMI 1640 supplemented with 10% FCS (RPMI/FCS) before use in flow cytometry or as effector cells in the cytotoxic assays.

**Detection of antibody-dependent cellular cytotoxicity in an *in vitro* model**

The antibody-dependent cellular cytotoxicity (ADCC) of the recombinant antibodies according to the invention was investigated in an europium release assay. Target cells (ZR-75-1, $5 \times 10^6$) were incubated for 6 minutes on ice in 800 $\mu$l of europium buffer (50 mM HEPES, pH 7.4, 93 mM NaCl, 5 mM KCl, 2 mM $MgCl_2$, 10 mM diethylenetri-aminepentaacetic acid, 2 mM europium(III)acetate), electroporated (710 V, 1 pulse, 30 $\mu$s) in a Multiporator (eppendorf), and subsequently incubated on ice for another 6 min. Thereafter, the cells were washed 5 times in RPMI 1640/5% FCS and seeded in a 96-well round-bottom plate (Nunc; $1 \times 10^4$ cells in 100 $\mu$l per well). Following addition of 20 $\mu$l of recombinant antibodies at varying concentration (0.5 to 25 $\mu$g/ml final concentration in 200 $\mu$l incubation volume) or the corresponding controls (medium, isotype control human IgG), human peripheral blood cells (80 $\mu$l per well) were added as effector cells, using an effector/target cell ratio of 50:1. 80 $\mu$l RPMI/FCS with no effector cells was added to determine spontaneous release. Maximum release was determined after complete lysis of the target cells with ethanol. Following incubation in an incubator at 37°C for 4 hours, the plate was centrifuged at 500 x g for 5 minutes, and 25 $\mu$l of each sample was pipetted in 200 $\mu$l per well of enhancement solution (Perkin-Elmer Wallac). Following incubation for 15 min at room temperature, the fluorescence was determined (Victor$^2$ Fluorometer, Perkin-Elmer Wallac). The specific cytotoxicity is obtained from the equation (experimental lysis - spontaneous lysis) / (maximum lysis - spontaneous lysis).

The ADCC activity of the chimaeric PankoMab isolated from NM-F9, K562, NM-H9, NM-D4, and NM-H9D8 is about 5 times higher than the ADCC activity of the chimaeric PankoMab isolated from the CHOdhfr- cells. About a fifth of the chimaeric PankoMab concentration leads to the same ADCC activity as the chimaeric PankoMab expressed in the CHOdhfr- cells.

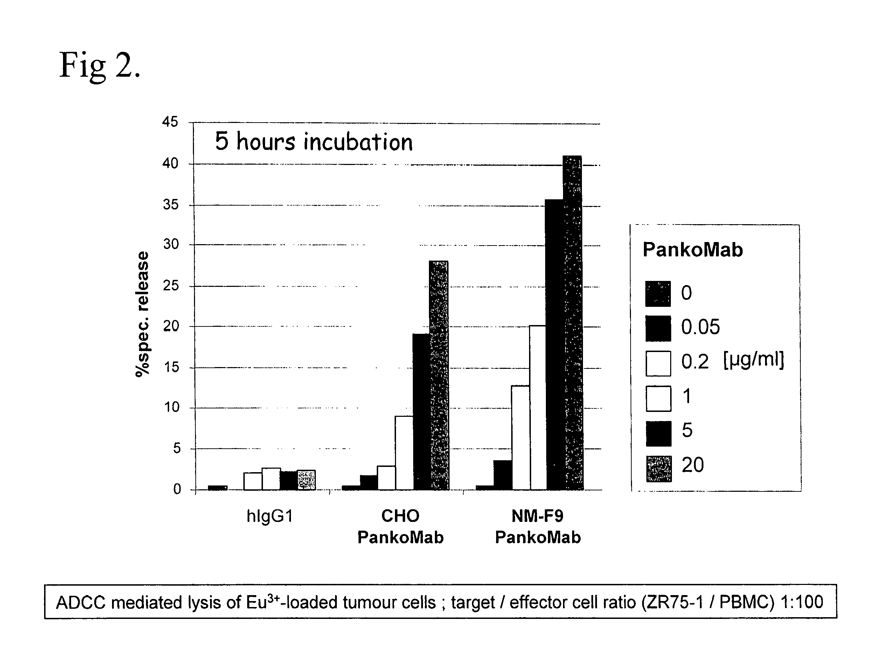

See also Fig.2.

**Detection of complement-dependent cellular cytotoxicity in an *in vitro* model**

The complement-dependent cellular cytotoxicity (CDC) of antibodies was investigated in an europium release assay. $Eu^{3+}$-loaded target cells (NM-D4, $5 \times 10^6$) were prepared as described above. Thereafter, the cells were seeded in a 96-well round-bottom plate (Nunc; $2 \times 10^4$ cells in 100 $\mu$l per well). Following addition of 20 $\mu$l of antibodies at varying concentration (0.5 to 50 $\mu$g/ml final concentration in 200 $\mu$l incubation volume) or the corresponding controls (medium, isotype control murine IgM), cells were incubated half an hour at room temperature. Thereafter, 10 $\mu$l per well baby rabbit complement (Cedarline, 1:5 to 1:10 diluted) were added. 10 $\mu$l RPMI/FCS with no complement was added to determine spontaneous release. Maximum release was determined after complete lysis of the target cells with ethanol. Following incubation in an incubator at 37°C for 3 to 3.5 hours, the plate was centrifuged at 500 x g for 5 minutes, and 25 $\mu$l of each sample was pipetted in 200 $\mu$l per well of enhancement solution (Perkin-Elmer Wallac). Following incubation for 15 min at room temperature, the fluorescence was determined (Victor$^2$ Fluorometer, Perkin-Elmer Wallac).

The specific cytotoxicity is obtained from the equation (experimental lysis - spontaneous lysis) / (maximum lysis - spontaneous lysis).

**Conjugate formation assay (CFA) to analyse phagocytosis activity of antibody molecule compositions**

*Tumor cell staining with PKH26:*

$1 \times 10^7$ to $2 \times 10^7$ tumor cells were washed twice in PBS, resuspended in 1ml diluent C, 1ml PKH26 was added at concentration $12 \times 10^{-6}$ M for ZR-74-1, ZR-74-1TF, MCF-7 and MT-3.

After 3min incubation at RT, staining was stopped by adding 2ml FCS for 1min at RT. Medium (4ml, RPMI supplemented with 1% L-glutamine, 10 % FCS) was added, cells were spun down and washed 4 times with medium. Tumor cells were incubated o.n. at 37°C, 5% $CO_2$ to allow the release of excess PKH-26.

Optimization of PKH-26 staining was performed with half of the cell numbers and volumes.

*CFA:*

PKH-26 labeled tumor cells were harvested with trypsin/EDTA, washed once with PBS and resuspended at $0.8 \times 10^6$ cells/ml in MAK cell medium (Invitrogen) in the presence or absence of recombinant antibody molecule compositions. Tumor cells (250$\mu$l, $0.2 \times 10^6$ cells) were seeded in non-adherent polypropylene tubes.

MAK cells were prepared according to Boyer et al., Exp. Hematol. 27, 751-761 (1999), washed and resuspended at $1.6 \times 10^6$ cells/ml. 250$\mu$l MAK cells ($0.2 \times 10^6$ cells) were added to PKH-26 labeled tumor cells (E:T ratio 2:1). Individual samples were incubated in duplicates at 4°C and 37°C/5% $CO_2$ for 3 h or o.n. (18 -20h).

After incubation at 3h or o.n. cells were washed with PBS and stained with CD11c-FITC (1:18,5) + 7-AAD (1: 500) in PBS/10% FCS. Cells were washed with PBS, resuspended in 400$\mu$l PBS. Acquisition was done on 10.000 cells in an Epics XL (Beckman Coulter).

Percentage of colocalized MAK cells was determined as percentage of PKH-26 positive cells in the cell population gated for all viable CD11c-FITC positive cells.

**Example 9**

**Analysis of antibody molecule composition binding activity in ELISA**

To analyse antigen binding of antibody molecule compositions expressed in different cell lines purified antibody molecule compositions of chimeric PankoMab were measured in an ELISA on synthetic glycosylated MUC1 30-mer peptide with the sequence APPAHGVTSAPDT [GalNAcα]RPAPGSTAPPAHGVTSA. Non-glycosylated MUC1 peptide served as a control.

Using stock solutions (1 mg in 1ml of bidest. $H_2O$) stored in portions at -20°C, a dilution of 1 $\mu$g/ml in PBS was produced. 50 $\mu$l/well was pipetted in a NUNC Immuno plate F96 MaxiSorp, and the test plate was incubated at 4°C overnight. On the next day, the test plate was washed 3 times with PBS/0.2% Tween-20. Subsequently, non-specific binding sites were blocked with 2% BSA in PBS, incubated for at least 1 h at room temperature, and 50 $\mu$l of the recombinant PankoMab was applied (1- 400 ng/ml PBS/1%BSA). After three wash steps with PBS/0.2% Tween-20, peroxidase-coupled secondary anti-human Fcγ1 antibody was employed to detect the specifically bound antibody. To detect the bound secondary antibody, a color reaction with TMB (3,3',5,5'-tetramethylbenzidine) was performed. After 15 minutes the reaction was quenched by adding 2.5 N $H_2SO_4$. Measurement was performed using a microtiter plate photometer with 450 nm filter in dual mode versus 630 nm reference filter.

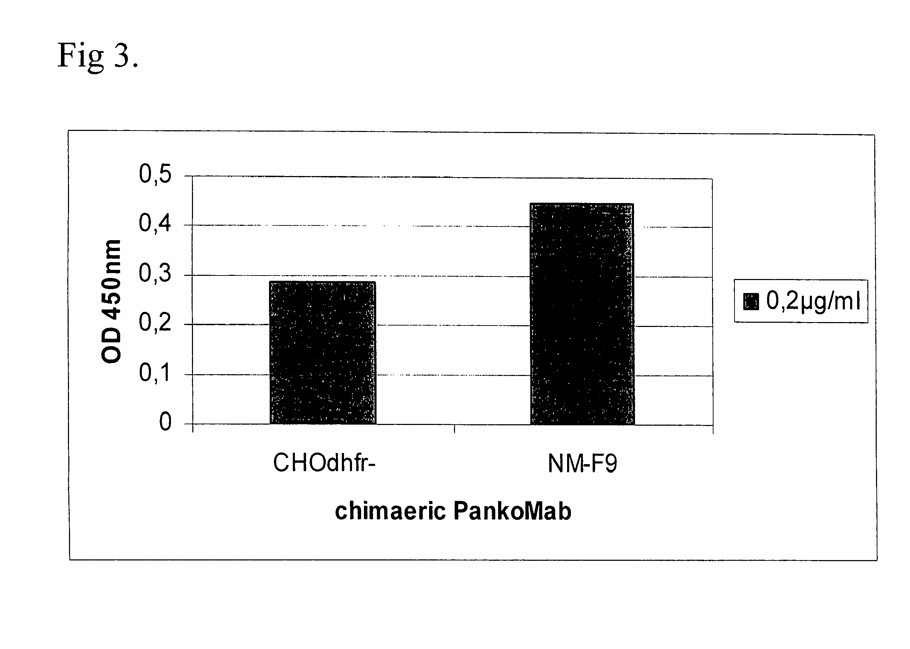

The binding activity of the chimaeric PankoMab isolated from NM-F9 is about 50% higher than the binding activity of the chimaeric PankoMab isolated from the CHOdhfr- cells (Fig.3).

**Example 10**

**Glycan analysis of antibody molecule compositions expressed in NM-F9 and CHOdhfr- cells**

Glycans of at least 100$\mu$g purified antibody were cleaved by acid hydrolysis. Sialic acids were labelled specifically by conjugation with the fluorescence dye DMB. Analysis was performed by HPLC e.g. on a Asahipak-NH2 column to separate the saccharides. Identification and calculation of sialic acids was performed by standard substances of appropriate sialic acids.

Analysis of chimeric PankoMab antibody molecule composition expressed in CHOdhfr- or NM-F9 cells revealed in <10% monosialylation of the CHOdhfr- product and no sialylation at all of the NM-F9 product. The latter contained only noncharged glycans.

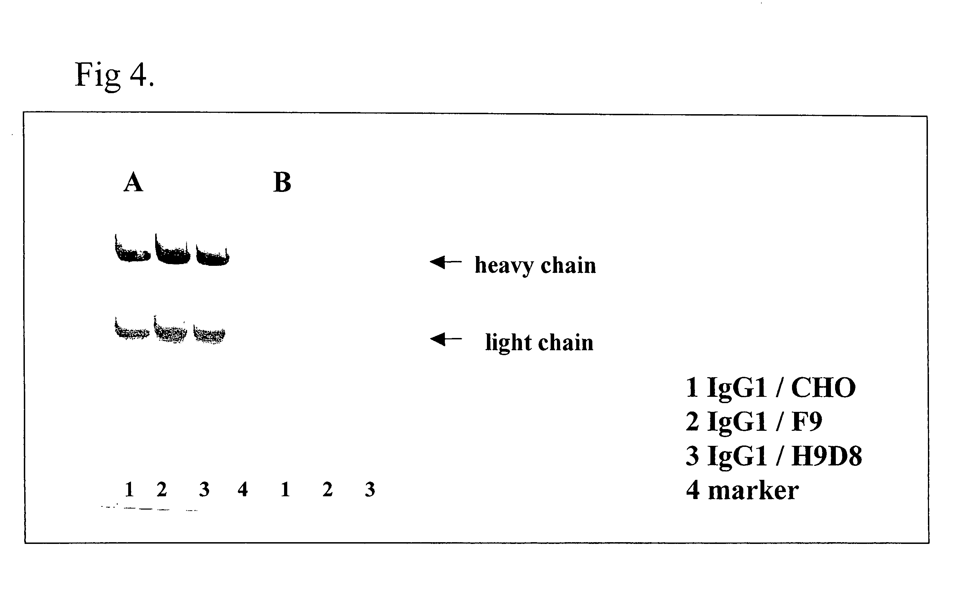

Western blot analysis was performed to identify the differently sialylated heavy chain of antibody molecule compositions expressed in CHOdhfr-, NM-F9, or NM-H9D8. 5$\mu$g of each antibody molecule composition were separated by SDS-Page in an 10% acrylamide gel under reducing conditions. Proteins were transfered to nitrocellulose and visualized either by secondary anti-human IgG antibodies (Fig. 4A) or SNA (Fig. 4B) which detects 2-6 sialylation.

It is to be understood that this invention is not limited to the particular methodology, protocols and/or reagents as described herein as these may vary. It is also to be understood that the terminology used herein is for the purpose of describing particular embodiments only, and is not intended to limit the scope of the present invention which will be limited only by the appended claims.

**Table 1**

| Yield of chimaeric PankoMab expressed in CHOdhfr- and NM-F9 cultured in medium supplemented with FCS | | |
|---|---|---|
| | **CHOdhfr-** | **NM-F9** |
| **Production rate (cIgG1)**<br><br>pcd determined after 1st to 4th round of gene amplification (methotrexate) and single cell cloning from theoretically 200 seeded clones (limited dilution) | 1st round (100nM MTX) Average: 2 +/- 1 pcd; Max: 3 pcd<br>2nd round (200nM MTX) Average: 5 +/- 1 pcd; Max: 7 pcd<br><br>3rd round (500nM MTX) Average: 9 +/- 5 pcd; Max: 17 pcd<br><br><br>4th round (1000nM MTX) Average: 13 +/- 5 pcd; Max: 22 pcd | 1st round (100nM MTX)<br>Average: 5 +/- 1,5 pcd; Max: 13 pcd<br>2nd round (200nM MTX)<br>Average: 7.5 +/- 4.5 pcd; Max: 19 pcd<br>3nd round (500nM MTX)<br>**Average: 12.5 +/- 4.5 pcd; Max:19 pcd**<br>4nd round (1000nM MTX)<br>**Average: 16.5 +/- 6 pcd; Max: 27 pcd** |

**Table 2**

| Yield of chimaeric PankoMab expressed in NM-H9D8 cultured in serum-free medium | |
| --- | --- |
| | **NM-H9D8** |
| **Production rate (cIgG1)**<br><br>pcd determined after 1st to 5th round of gene amplification (methotrexate) and single cell cloning from theoretically 200 seeded clones (limited dilution) | 1st round (100nM MTX)<br>Average: 7 +/- 4 pcd; Max: 14 pcd<br>2nd round (200nM MTX)<br>Average: 14 +/- 4 pcd; Max: 23 pcd<br>3rd round (500nM MTX)<br>Average: 13 +/- 6 pcd; Max: 31 pcd<br>4th round (1000nM MTX)<br>Average: 23 +/- 8 pcd; Max: 34 pcd<br>5th round (2000nM MTX)<br>Average: 23 +/- 9 pcd; Max: 38 pcd |

**Claims**

1. A method for producing an antibody molecule composition, comprising:

   (a) introducing in a host cell of human myeloid leukaemia origin at least one nucleic acid encoding an antibody molecule or at least one part thereof, and at least one nucleic acid comprising at least one nucleic acid sequence encoding at least one polypeptide of the group of sequence #1 to sequence #9; and
   (b) amplifying the nucleic acid sequence encoding said antibody molecule or at least one part thereof by culturing said host cell with methotrexate; and
   (c) culturing said host cell under conditions which permits the production of said antibody molecule composition, and
   (d) isolating said antibody molecule composition.

2. A method for producing an antibody molecule composition

   (i) having an increased Fc-mediated cellular cytotoxicity which is at least 2 times higher than the Fc-mediated cellular cytotoxicity of at least one antibody molecule composition from the same antibody molecule when expressed in the cell line CHOdhfr- [ATCC No. CRL-9096];
   and/or
   (ii) having an increased antigen mediated or Fc-mediated binding which is at least 50% higher than the binding of at least one antibody molecule composition from the same antibody molecule when expressed in the cell line CHOdhfr- [ATCC No. CRL-9096];
   and/or
   (iii) having an increased average or maximum yield of said antibody molecule composition which is at least 10% higher than the yield of at least one antibody molecule composition from the same antibody molecule when expressed in the cell line CHOdhfr- [ATCC No. CRL-9096];
   and/or
   (iv) comprising no detectable NeuGc;
   and/or
   (v) comprising no detectable NeuNAc;
   and/or
   (vi) comprising detectable alpha2-6 linked NeuNAc;
   and/or
   (vii) having a higher sialylation degree with at least a 15% higher amount of N-acetylneuraminic acid on the total carbohydrate units or on at least one particular carbohydrate chain at a particular glycosylation site of the antibody molecule of the antibody molecules in said antibody molecule composition than the same amount of antibody molecules of at least one antibody molecule composition of the same antibody molecule isolated from CHOdhfr- [ATCC No. CRL-9096] when expressed therein,

   comprising:

(a) introducing in a host cell of human myeloid leukaemia origin at least one nucleic acid encoding an antibody molecule or at least one part thereof; and
(b) culturing said host cell under conditions which permits the production of said antibody molecule composition; and
(c) isolating said antibody molecule composition.

**3.** A method for producing an antibody molecule composition

vii. having an increased Fc-mediated cellular cytotoxicity which is at least 2 times higher than the Fc-mediated cellular cytotoxicity of at least one antibody molecule composition from the same antibody molecule when expressed in the cell line CHOdhfr-[ATCC No. CRL-9096];
and/or
viii. having an increased antigen mediated or Fc-mediated binding which is at least 50% higher than the binding of at least one antibody molecule composition from the same antibody molecule when expressed in the cell line CHOdhfr- [ATCC No. CRL-9096];
and/or
ix. having an increased average or maximum yield of said antibody molecule composition which is at least 10% higher than the yield of at least one antibody molecule composition from the same antibody molecule when expressed in the cell line CHOdhfr- [ATCC No. CRL-9096];
and/or
x. comprising no detectable NeuGc;
and/or
xi. comprising no detectable NeuNAc;
and/or
xii. comprising detectable alpha2-6 linked NeuNAc;
and/or
xiii. having a higher sialylation degree with at least a 15% higher amount of N-acetylneuraminic acid on the total carbohydrate units or on at least one particular carbohydrate chain at a particular glycosylation site of the antibody molecule of the antibody molecules in said antibody molecule composition than the same amount of antibody molecules of at least one antibody molecule composition of the same antibody molecule isolated from CHOdhfr- [ATCC No. CRL-9096] when expressed therein,

comprising:

(e) introducing in a host cell of human myeloid leukaemia origin at least one nucleic acid encoding an antibody molecule or at least one part thereof, and at least one nucleic acid comprising at least one nucleic acid sequence encoding at least one polypeptide of the group of sequence #1 to sequence #9; and
(f) amplifying the nucleic acid sequence encoding said antibody molecule or at least one part thereof by culturing said host cell with methotrexate; and
(g) culturing said host cell under conditions which permits the production of said antibody molecule composition; and
(h) isolating said antibody molecule composition.

**4.** The method of claim 1 to 5, wherein the host cell of human myeloid leukaemia origin of the invention grows and produces the antibody molecule composition under serum-free conditions.

**5.** The method of claim 6, wherein the nucleic acid encoding the antibody molecule is introduced and isolated under serum-free conditions.

**6.** The method of claim 1. to 5., wherein in step (a), said host cell is K562, NM-F9 [DSM ACC2606], NM-D4 [DSM ACC2605], NM-H9, H9, NM-H10, NM-E-2F9, NM-C-2F5, NM-H9D8 [DSM ACC], or NM-H9D8-E6, or a cell or cell line derived from anyone of said host cells.

**7.** A host cell of human myeloid leukaemia origin comprising at least one nucleic acid encoding an antibody molecule or at least one part thereof, and at least one nucleic acid comprising at least one nucleic acid sequence encoding at least one polypeptide of the group of sequence #1 to sequence #9

**8.** The host cell K562, NM-F9 [DSM ACC2606], NM-D4 [DSM ACC2605], NM-H9, H9, NM-H10, NM-E-2F9, NM-C-2F5, NM-H9D8 [DSM ACC], or NM-H9D8-E6, or a cell or cell line derived from anyone of said host cells, comprising at least one nucleic acid encoding an antibody molecule or at least one part thereof, and at least one nucleic acid comprising at least one nucleic acid sequence encoding at least one polypeptide of the group of sequence #1 to sequence #9.

9. A host cell of any of the claims 7 to 8, wherein the antibody molecule encoded is an antibody of WO2004/065423

10. A host cell of any of the claims 7 to 8, wherein the antibody molecule encoded is a chimearic or humanized PankoMab

11. An antibody composition produced or a molecule isolated from the antibody composition produced by any of the claims 1 to 6.

Fig 1.

# Fig 2.

5 hours incubation

PankoMab

- 0
- 0.05
- 0.2 [µg/ml]
- 1
- 5
- 20

ADCC mediated lysis of Eu$^{3+}$-loaded tumour cells ; target / effector cell ratio (ZR75-1 / PBMC) 1:100

Fig 3.

OD 450 nm

0,5 0,4 0,3 0,2 0,1 0

CHOdhfr-    NM-F9

chimaeric PankoMab

■ 0,2 µg/ml

Fig 4.

A       B

← heavy chain

← light chain

1  2   3   4   1   2   3

1 IgG1 / CHO
2 IgG1 / F9
3 IgG1 / H9D8
4 marker

**European Patent Office**

**EUROPEAN SEARCH REPORT**

Application Number

EP 06 09 0190

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | KANEKO Y ET AL: "ANTI-INFLAMMATORY ACTIVITY OF IMMUNOGLOBULIN G RESULTING FROM FC SIALYLATION" SCIENCE, AMERICAN ASSOCIATION FOR THE ADVANCEMENT OF SCIENCE,, US, vol. 313, no. 5787, August 2006 (2006-08), pages 670-673, XP008076047 ISSN: 0036-8075 * page 671, column 2, paragraph 1 - column 3, paragraph 2; figures 1,2 * | 2,3 | INV. C07K16/00 C07K16/30 C12N5/10 |
| X | * page 671, column 2, paragraph 1 - column 3, paragraph 2; figures 1,2 * ----- | 11 | |
| X | GOLETZ S: 9 April 2006 (2006-04-09), XP002423302 Turning Glycomics Into Health Retrieved from the Internet: URL:http://www.invest-in-germany.de/upload _files/20060425091024_Praesentation_GLYCOT OPE_German_Country_Presentation.pdf> * page 8 * ----- | 11 | |
| Y | WO 2005/080585 A (GLYCOTOPE GMBH [DE]; GOLETZ STEFFEN [DE]; BAUMEISTER HANS [DE]; SCHOEB) 1 September 2005 (2005-09-01) * page 14, line 30 - page 15, line 10 * * figures 11-15 * * page 28, line 20 - line 24 * * page 35, line 18 - line 24 * * page 41, line 9 - line 14 * ----- | 1-10 | TECHNICAL FIELDS SEARCHED (IPC) C07K |
| Y | EP 0 117 060 A (GENENTECH INC [US]) 29 August 1984 (1984-08-29) * page 4, line 26 - page 5, line 2 * * figure 1b * * claim 16 * ----- -/-- | 1,3-10 | |

The present search report has been drawn up for all claims

| Place of search | Date of completion of the search | Examiner |
|---|---|---|
| The Hague | 24 April 2007 | Cilensek, Zoran |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 1 911 766 A1**

European Patent Office

**EUROPEAN SEARCH REPORT**

Application Number

EP 06 09 0190

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DORAI H ET AL: "The effect of dihydrofolate reductase-mediated gene amplification on the expression of transfected immunoglobulin genes." JOURNAL OF IMMUNOLOGY (BALTIMORE, MD. : 1950) 15 DEC 1987, vol. 139, no. 12, 15 December 1987 (1987-12-15), pages 4232-4241, XP002427555 ISSN: 0022-1767 * the whole document * | 1-11 | |
| A | EP 1 167 537 A1 (JAPAN TOBACCO INC [JP]; ABGENIX INC [US]) 2 January 2002 (2002-01-02) * line 35 - page 4, line 38 * * page 10, line 50 - line 57 * * claim 13 * | 1-11 | |
| A | WO 2005/017130 A (GLYCOTOPE GMBH [DE]; GOLETZ STEFFEN [DE]; BAUMEISTER HANS [DE]; SCHLAN) 24 February 2005 (2005-02-24) * the whole document * | 1-11 | **TECHNICAL FIELDS SEARCHED (IPC)** |
| A | DATABASE Geneseq [Online] 17 November 2005 (2005-11-17), "DHFR-synuclein fusion protein GST-ATSalpha SEQ ID NO:81." XP002430726 retrieved from EBI accession no. GSP:AEC80429 Database accession no. AEC80429 * the whole document * | 1-11 | |

-/--

The present search report has been drawn up for all claims

| Place of search | Date of completion of the search | Examiner |
|---|---|---|
| The Hague | 24 April 2007 | Cilensek, Zoran |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent
Office

**EUROPEAN SEARCH REPORT**

Application Number

EP 06 09 0190

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DATABASE EPO Proteins [Online] 10 March 2005 (2005-03-10), "Sequence 628 from Patent WO2005016962." XP002430727 retrieved from EBI accession no. EPOP:CS031122 Database accession no. CS031122 * the whole document * ----- | 1-11 | |

TECHNICAL FIELDS
SEARCHED (IPC)

The present search report has been drawn up for all claims

| Place of search | Date of completion of the search | Examiner |
|---|---|---|
| The Hague | 24 April 2007 | Cilensek, Zoran |

EPO FORM 1503 03.82 (P04C01)

## ANNEX TO THE EUROPEAN SEARCH REPORT
## ON EUROPEAN PATENT APPLICATION NO.

EP 06 09 0190

This annex lists the patent family members relating to the patent documents cited in the above-mentioned European search report.
The members are as contained in the European Patent Office EDP file on
The European Patent Office is in no way liable for these particulars which are merely given for the purpose of information.

24-04-2007

| Patent document cited in search report | | Publication date | Patent family member(s) | | Publication date |
|---|---|---|---|---|---|
| WO 2005080585 | A | 01-09-2005 | AU | 2005215889 A1 | 01-09-2005 |
| | | | CA | 2557725 A1 | 01-09-2005 |
| EP 0117060 | A | 29-08-1984 | AU | 2353384 A | 26-07-1984 |
| | | | DK | 21084 A | 24-08-1984 |
| | | | ES | 8600780 A1 | 01-02-1986 |
| | | | JP | 59192089 A | 31-10-1984 |
| | | | ZA | 8400376 A | 27-02-1985 |
| EP 1167537 | A1 | 02-01-2002 | AU | 754808 B2 | 28-11-2002 |
| | | | AU | 3456300 A | 16-10-2000 |
| | | | CA | 2368734 A1 | 05-10-2000 |
| | | | WO | 0058499 A1 | 05-10-2000 |
| | | | US | 2006059575 A1 | 16-03-2006 |
| WO 2005017130 | A | 24-02-2005 | US | 2006292129 A1 | 28-12-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6946292 B
- WO 0061739 A
- US 6602684 B
- WO 2005017130 A2
- US 20030115614 A1
- WO 2005080585 A1
- WO 2004065423 A
- WO 2004050707 A
- EP 1654353 A

### Non-patent literature cited in the description

- **ROYSTON JEFFERIS.** CCE IX: Glycosylation of Recombinant Antibody Therapeutics. *Biotechnol.Prog.,* 2005, vol. 21, 11-16
- **BOYD et al.** *Molecular Immunol,* 1995, vol. 32, 1311
- **LIFELY et al.** *Glycobiology,* December 1995, vol. 5 (8), 813-22
- **UMANA et al.** *Nature Biotechn,* 1999, vol. 17, 176-180
- **YOSHIMURA M et al.** *Cancer Res.,* 1996, vol. 56 (2), 412-8
- *Cancer Immunol Immunother,* November 2006, vol. 55 (11), 1337-47
- **BOYER et al.** *Exp. Hematol.,* 1999, vol. 27, 751-761